# EUROPEAN PATENT SPECIFICATION

(11) **EP 3 601 605 B1**
(45) Date of publication and mention of the grant of the patent: **22.11.2023**
(21) Application number: 18770338.4
(22) Date of filing: 23.03.2018
(51) Int. Cl.: C12Q 1/6804, G01N 33/50, C12Q 1/686, G01N 33/53

(54) **METHODS FOR MULTIPLEX DETECTION OF MOLECULES**
VERFAHREN ZUR MULTIPLEX-DETEKTION VON MOLEKÜLEN
PROCÉDÉS DE DÉTECTION MULTIPLEX DE MOLÉCULES

(30) Priority: 24.03.2017 SG 10201702423X
(43) Date of publication of application: 05.02.2020
(73) Proprietor: National University of Singapore, Singapore 119077 (SG); Agency for Science, Technology and Research, Singapore 138632 (SG)
(72) Inventor: SCOLNICK, Jonathan, Singapore 119077 (SG); YEO, Eugene, Singapore 119077 (SG); HOON, Shawn, Singapore 138673 (SG)
(74) Representative: De Clercq & Partners
(86) International application number: PCT/SG2018/050130
(87) International publication number: WO 2018/174827

(56) References cited:
- WO-A1-01/09376
- WO-A1-2015/031691
- WO-A1-2016/011364
- WO-A1-2016/123419
- WO-A1-2016/123419
- WO-A1-2016/145409
- WO-A1-2017/053905
- WO-A1-2017/053905
- WO-A1-2018/057051
- WO-A2-2013/102108
- WO-A2-2013/102108
- WO-A2-2016/145416
- WO-A2-2017/124101

## Description

### RELATED APPLICATION(S)

### BACKGROUND OF THE INVENTION

The detection and quantification of proteins is important for both molecular biology research and medical applications. Immuno-PCR techniques provide one method by which proteins with DNA labels in a variety of different formats can be detected. Many immuno-PCR approaches use heterogeneous assays, which involve initial immobilization of a target analyte to a surface with subsequent detection using an antibody with a DNA label and oligonucleotide tags (IDs) for amplification purposes. Although these approaches represent notable advances in protein detection, they have several drawbacks including low sensitivity and slow target binding kinetics. Further, they require complex conjugation chemistries.

WO2016123419 A1 relates to methods of imaging molecules without a microscope or other specialized equipment, but using molecular instruments (e.g., DNAbased and protein-based molecules).

WO2013102108 A2 relates to compositions of nucleic acid hybridization probes for detecting a nucleic acid target sequence and methods for their production.

WO2017053905 A1 and WO2018057051 A1, each being a document under Art. 54(3) EPC, relate to methods and composition for single cell characterization using affinityoligonucleotide conjugates.

A sensitive and rapid method for detecting non-DNA molecules in a sample is needed.

### SUMMARY OF THE INVENTION

The present invention provides methods, binding agents and kits for quantifying elements or components of a sample. For example, the methods may be applied to the quantification of proteins, nucleic acids and/or cells present in a sample.

In some embodiments, the present disclosure provides methods which may be used to obtain proteomic and nucleic acid information from samples.

The invention provides a method of detecting a target non-nucleic acid molecule (e.g., a target protein) and a target nucleic acid molecule (e.g., a target mRNA) in a sample. The method generally comprises the steps of:
a) contacting a sample comprising a target non-nucleic acid molecule and a target nucleic acid molecule with a binding agent under conditions that permit binding between the binding agent and the target non-nucleic acid molecule in the sample, wherein the binding agent is covalently conjugated to an oligonucleotide that comprises a binding agent identification tag and a poly(A) sequence;
b) reverse transcribing the conjugated oligonucleotide and the target nucleic acid molecule in the sample simultaneously, thereby producing a conjugated oligonucleotide reverse transcription product and a target nucleic acid molecule reverse transcription product, wherein reverse transcription of the conjugated oligonucleotide is primed using an extension primer that comprises a poly(dT) sequence, wherein the length of the poly(dT) sequence is greater than or equal to the length of the poly(A) sequence in the conjugated oligonucleotide;
c) amplifying the conjugated oligonucleotide reverse transcription product and the target nucleic acid molecule reverse transcription product simultaneously, thereby producing a conjugated oligonucleotide amplicon and a target nucleic acid molecule amplicon; and
d) detecting: 1) the conjugated oligonucleotide amplicon, thereby detecting the target non-nucleic acid molecule in the sample; and 2) the target nucleic acid molecule amplicon, thereby detecting the target nucleic molecule in the sample.

The methods of the invention can for example be performed using kits or binding agents as described below. In particular embodiments, the binding agent is covalently conjugated to an oligonucleotide that comprises a blocking group that prevents extension of the primer by a polymerase. In some embodiments, the binding agent comprises a binding agent identification tag. In certain embodiments, the binding agent is an antibody.

The kit generally comprises a binding agent that is covalently conjugated to an oligonucleotide, and one or more DNA amplification primers. The conjugated oligonucleotide further comprises a binding agent identification tag.

In particular embodiments, the invention provides a method of detecting a target protein and a target nucleic acid molecule in a sample. The method generally comprises the steps of:
a) contacting a sample comprising a target protein and a target nucleic acid molecule with a binding agent under conditions that permit binding between the binding agent and the target protein in the sample, wherein the binding agent is covalently conjugated to an oligonucleotide that comprises a binding agent identification tag and a poly(A) sequence;
b) reverse transcribing the conjugated oligonucleotide and the target nucleic acid molecule in the sample simultaneously, thereby producing a conjugated oligonucleotide reverse transcription product and a target nucleic acid molecule reverse transcription product, wherein reverse transcription of the conjugated oligonucleotide is primed using an extension primer that comprises a poly(dT) sequence, wherein the length of the poly(dT) sequence is greater than or equal to the length of the poly(A) sequence in the conjugated oligonucleotide;
c) amplifying the conjugated oligonucleotide reverse transcription product and the target nucleic acid molecule reverse transcription product simultaneously, thereby producing a first conjugated oligonucleotide amplicon and a first target nucleic acid molecule amplicon;
d) separating the first conjugated oligonucleotide amplicon and the first target nucleic acid molecule amplicon;
e) amplifying the separated conjugated oligonucleotide amplicon and target nucleic acid molecule amplicon in separate reactions, thereby producing a second conjugated oligonucleotide amplicon and a second target nucleic acid molecule amplicon; and
f) detecting: 1) the second conjugated oligonucleotide amplicon, thereby detecting the target protein in the sample; and 2) the second target nucleic acid molecule amplicon, thereby detecting the target nucleic molecule in the sample.

The methods described herein provide certain advantages over existing methods, as proteomic and genomic information may be obtained simultaneously and from the same (or a single) sample. In addition, the methods allow a simple workflow for measuring both protein(s) and nucleic acid(s) from the same sample and without the need to split the sample. As described herein, the methodsof the invention can provide information (e.g., expression information) about numerous different target proteins and nucleic acids in a given sample.

### BRIEF DESCRIPTION OF THE DRAWINGS

The present invention will now be described in detail with reference to the following figures which show:

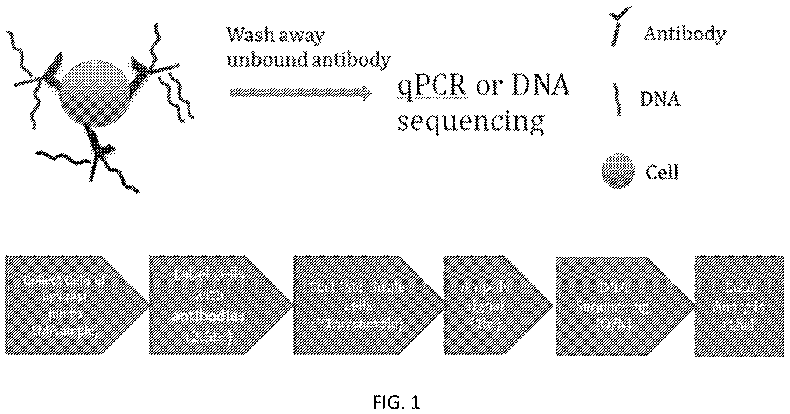

FIG. 1: A cell (circle) or group of cells is exposed to DNA conjugated antibodies. Three different antibodies shown here each have different DNA tags. The sample is washed to remove any unbound targeting molecules and the DNA labels are quantitated either by quantitative PCR or by high throughput DNA sequencing analysis.

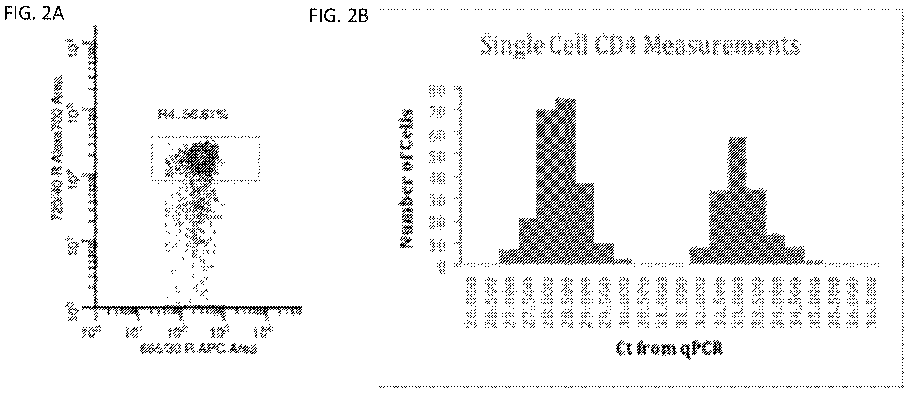

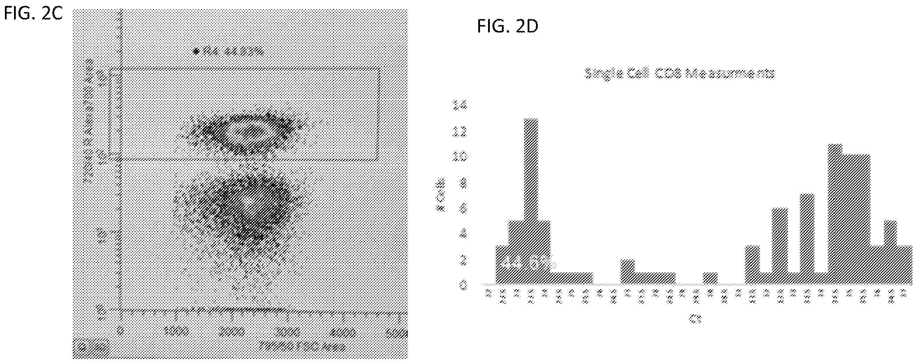

FIG. 2A is FACS plot showing 56.61% of CD3 positive cells also stained positive for CD4. FIG. 2B is a histogram of Ct values based on DNA tagged anti-CD4 shows that 58% of CD3 positive cells were labelled for CD4 using the DNA tagging method. FIG. 2C shows distribution of cells stained for CD8. FIG. 2D shows qPCR results from CD8 sorting.

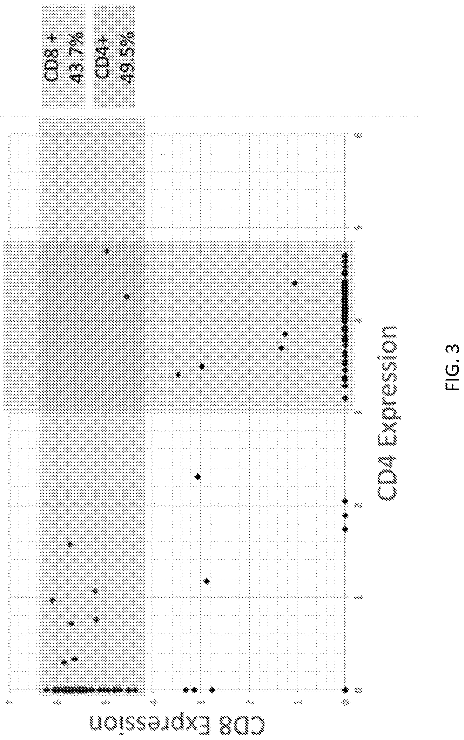

FIG. 3: Triple labelling of PBMCs with fluorescent CD3, and DNA barcoded CD4 and CD8. Results show that most cells expressing CD8 have undetectable signal for CD4 and vice versa. Low numbers of CD4, CD8 double positive cells do exist and two were captured here (overlap region). Numbers are a relative expression based on the inverse of the Ct. Undetectable PCR signal was set to Ct of 40 which is shown in this graph as zero signal.

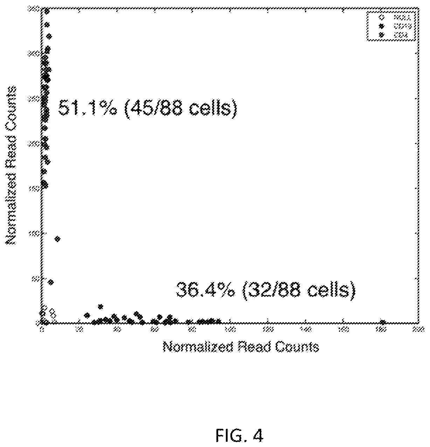

FIG. 4: Sequencing read counts show low background signal between the mutually exclusive expression of CD19 (B-cells) and CD4 (T cells).

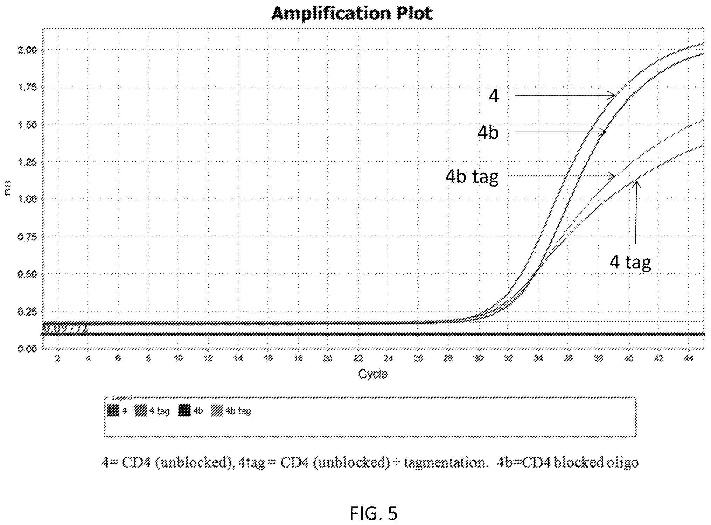

FIG. 5: An amplification plot.

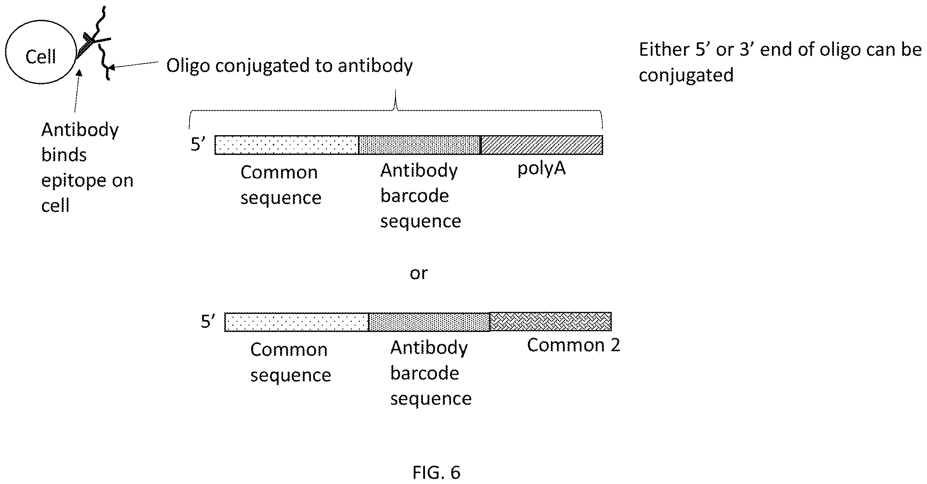

FIG. 6: Diagram depicting examples of conjugated oligonucleotides that are suitable for use in the methods described herein, including targeted RNA sequencing ("TCR") methods.

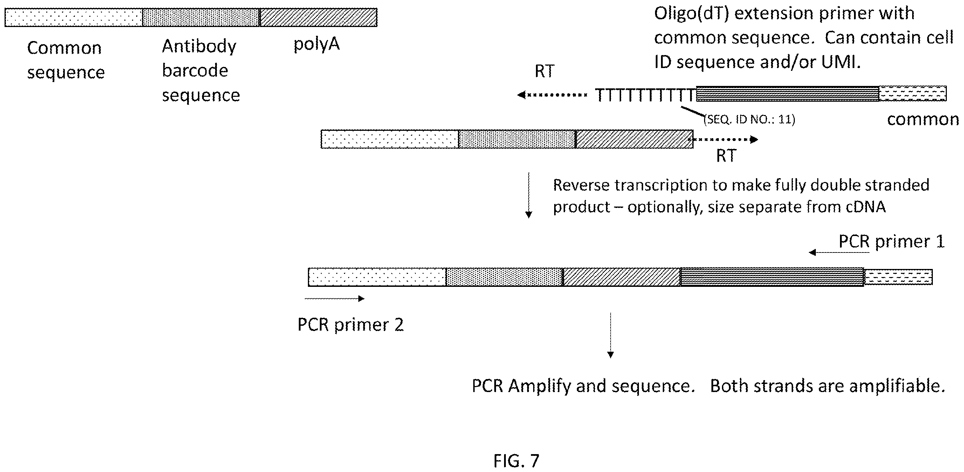

FIG. 7: Diagram depicting an example method of the invention, which includes a 5'-conjugated oligonucleotide containing a polyA tail (upper left) and an extension primer containing a cell identification (cell ID) tag and/or UMI and a dT tail (upper right) to prime RT of the conjugated oligonucleotide. During the cDNA amplification step (3' RACE), which uses PCR primer 1 only, a linear amplification of the RT product, which includes an antisense primer 1 binding site from the original conjugate strand, is obtained.

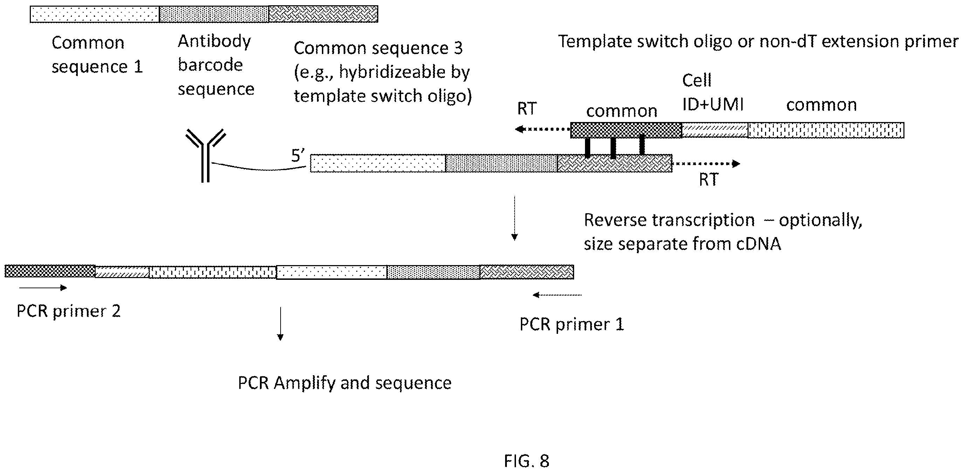

FIG. 8: Diagram depicting an example method of the invention, which includes a 5'-conjugated oligonucleotide that lacks a polyA tail (upper left) and a non-oligo(dT), or template switch, extension primer containing a cell identification (cell ID) tag and/or UMI (upper right) to prime RT of the conjugated oligonucleotide. In parallel, standard or targeted cDNA libraries can be generated. Both targeted 3' and targeted 5' RACE can be performed on the cDNA using gene specific primers.

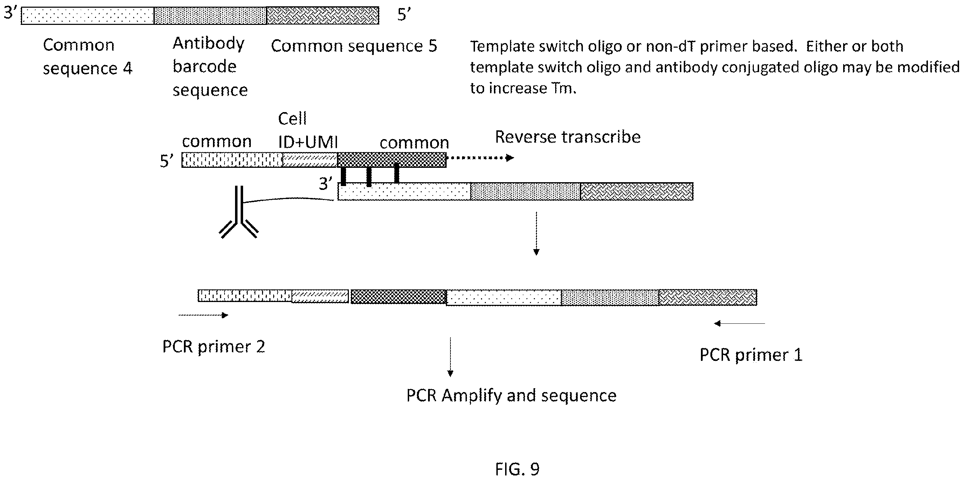

FIG. 9: Diagram depicting an example method of the invention, which includes a 3'-conjugated oligonucleotide that lacks a polyA tail (upper left) and a non-oligo(dT), or template, switch extension primer containing a cell identification (cell ID) tag and/or UMI (upper right) to prime RT of the conjugated oligonucleotide. In parallel, standard or targeted cDNA libraries can be generated. Both targeted 3' and targeted 5' RACE can be performed on the cDNA using gene specific primers.

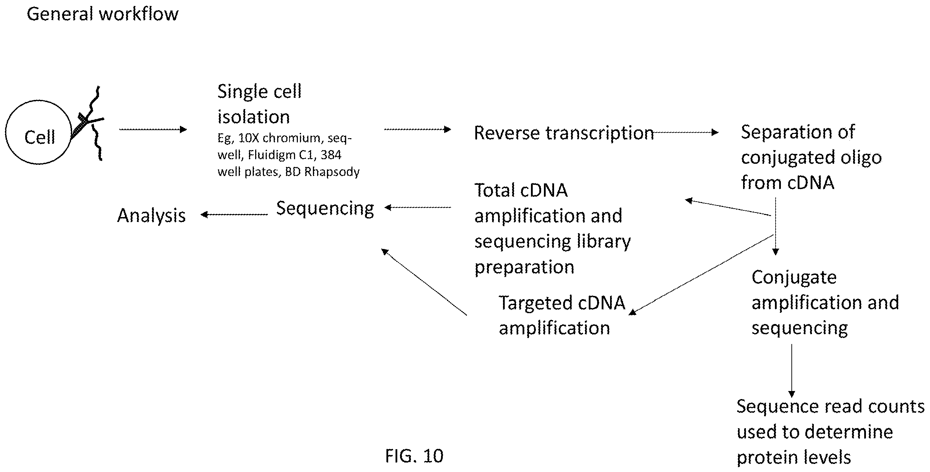

FIG. 10: General workflow diagram for an example method of the invention. While the antibody conjugates are being processed, cDNA can also be amplified in the same sample, for example, by dT priming and template switching (e.g., 5' RACE), or by gene specific primer RT priming with the cell ID placed on either the RT primer (3' end) or brought in later via a 5' primer. Each antibody can have a unique antibody barcode sequence. During the RT step, a unique molecular identifying (UMI) sequence can also be added, so that each DNA molecule receives a unique tag. Sequencing reads derived from the antibody conjugates are mapped to a list of known antibody barcodes. Reads are then collapsed based on the UMI; any sequences with the same antibody barcode and same UMI are only counted one time. Following the collapse of the data, the number of counts remaining for each antibody barcode is representative of the expression level of the target protein.

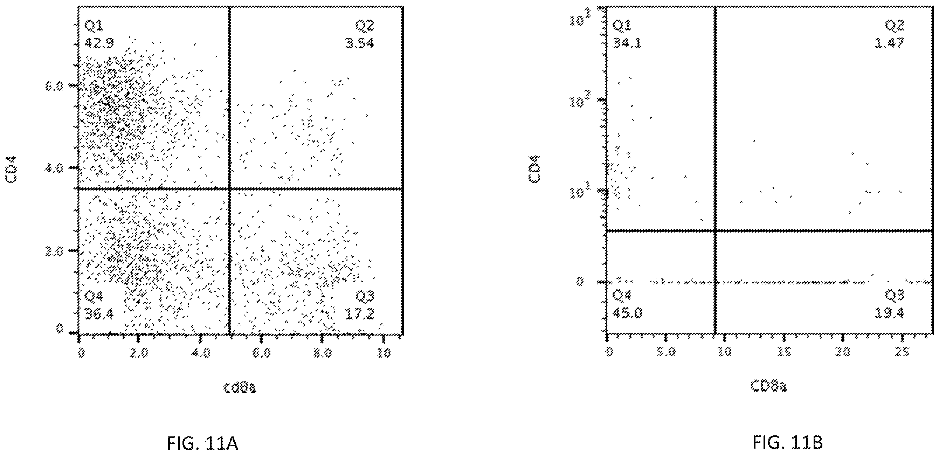

FIG. 11A: Protein expression data from a 3' RACE method using 10X genomics 3' RNA seq kit. Following the example method depicted in FIG. 8, PBMCs were stained with DNA encoded antibodies and used as input into the 10X Genomics Single Cell 3' reagent kit. Following library generation, antibody conjugated DNA was sequenced and read counts of CD4 and CD8 antibodies were plotted.
FIG. 11B: Protein expression data from a 5' RACE method using 10X genomics VDJ kit. Following the example method depicted in FIG. 8, PBMCs were stained with DNA encoded antibodies and used as input into the 10X Genomics Single Cell V(D)J reagent kit. Following library generation, antibody conjugated DNA was sequenced and read counts of CD4 and CD8 antibodies were plotted.

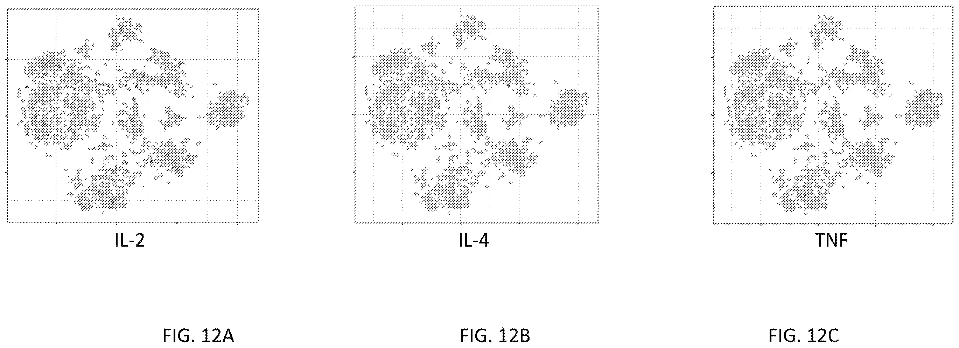

FIGS. 12A-12C: Human PBMCs cultured overnight either with or without addition of PMA to induce cytokine production (IL-2, IL-4 or TNF). Cells were combined and stained with DNA encoded antibodies according to method 1 prior to being used as input into the 10X Genomics Single Cell 3' Kit for library generation and sequencing. T-sne analysis showing gene expression data (RNA) projected onto cells (represented by dots) that have been clustered based on protein data. The dark spots are cells positive for gene expression of a given cytokine. The lighter dots are negative for that cytokine . Cells were treated with PMA for 1hr prior to staining/sequencing

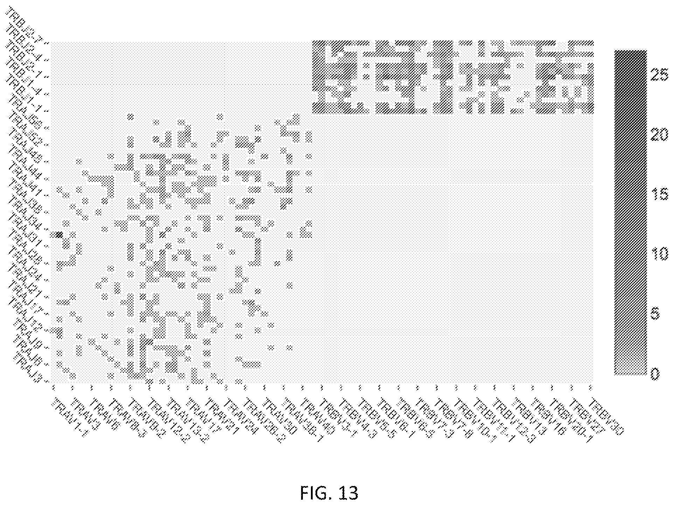

FIG. 13: TCR data using a 5' RACE technique. These TCRs found in the same cells as shown in the 5' RACE protein plots before. Following the example method depicted in FIG. 8, PBMCs were stained with DNA encoded antibodies and used as input into the 10X Genomics Single Cell V(D)J reagent kit. Following library generation, antibody conjugated DNA was sequenced and read counts of CD4 and CD8 antibodies were plotted. Additionally, the RNA samples were carried through the kit protocol to obtain T cell receptor sequences. The usage of VDJ segments in the alpha and beta subunits are plotted here using 10X Genomics software.

### DETAILED DESCRIPTION OF THE INVENTION

A description of example embodiments follows.

Unless defined otherwise, all technical and scientific terms used herein have the same meaning as commonly understood by one of ordinary skill in the art to which this invention pertains.

It should be noted that the term "proteomic" embraces any information relating to proteins present within a sample. For example the term may embrace quantity data relating to the number of proteins within a sample. The term may further (or also) relate to the type or identity of proteins present in a sample. One of skill will appreciate that where the protein is a protein expressed by a cell - by determining the quantity of such a cell associated protein in a sample, the number of cells can also be counted (the number of cell associated proteins being directly proportional to the number of cells present in the sample).

It should be noted that throughout this specification the term "comprising" is used to denote that embodiments of the invention "comprise" the noted features and as such, may also include other features. However, in the context of this invention, the term "comprising" may also encompass embodiments in which the invention "consists essentially of' the relevant features or "consists of" the relevant features.

In the context of this invention the term "nucleic acid" relates to the total nucleic acid content of a sample being analysed. For example the term nucleic acid embraces genomic nucleic acids (DNA and RNA) and other forms of nucleic acid such as plasmid nucleic acid and/or mitochondrial nucleic acid. Throughout this specification the term "genomic nucleic acid" is used - this term may relate to samples which comprise cells (for example mammalian, plant, bacterial or fungal cells) and relates to the total nucleic acid content including genomic DNA, RNA, plasmids, integrated viral nucleic acid, mitochondrial nucleic acid (DNA) and the like. Where the sample comprises viral particles, the term "nucleic acid" may apply to any type of nucleic acid associated with the virus - including any genomic DNA or RNA.

The term "nucleotide" refers to naturally occurring ribonucleotide or deoxyribonucleotide monomers, as well as non-naturally occurring derivatives and analogs thereof. Accordingly, nucleotides can include, for example, nucleotides comprising naturally occurring bases (*e.g.,* A, G, C, or T) and nucleotides comprising modified bases (*e.g*., 7-deazaguanosine, or inosine).

The term "sequence" or "polynucleotide sequence" in reference to a nucleic acid, refers to a contiguous series of nucleotides that are joined by covalent bonds (e.g., phosphodiester bonds).

The term "sample" embraces samples which comprise cells of all types. The cells may be living, viable or dead. The sample can be a cell or tissue sample, or a blood sample. The term "sample" also encompasses cell lysates and cell extracts.

The cells may be fixed.

Further, the term "cells" embraces mammalian cells, plant cells, bacterial cells and fungal cells.

As stated, a sample may comprise viral particles.

A sample may contain multiple cells (a plurality of cells) or a single cell.

In some embodiments, the sample comprises cellular components, but lacks whole or intact cells. For example, the sample can contain one or more types of cellular bodies, such as, e.g., nuclei, endosomes, mitochondria, or exosomes, or any combination thereof. In particular embodiments, the sample comprises exosomes.

The sample can also be an acellular sample or solution.

The sample can be placed in a well (e.g., microwell or nanowell), for example, on a plate or in an array. The well can further contain oligonucleotides (e.g., primers), which can be immobilized on beads.

The sample can be placed in a microdroplet (e.g., a droplet having volume of about 1 pL to about 10 nL), for example, for evaluation using a microfluidic-based platform.

Indeed the methods of this invention may be applied to almost any sample which represents a source of protein and nucleic acid. Ideally the protein and the nucleic acid are provided by a single source. One of skill will appreciate that where the sample comprises cells, each cell may represent a source of both protein and nucleic acid.

The methods described herein may find various applications and may be used to quantify, assay, determine, count, assess or measure some aspect of the sample.

As stated, the sample may comprise cells and as such the methods described herein may be exploited in order to determine information about the number or type of cells within a sample and/or the frequency of one or more cell marker types and information related to the total nucleic acid content of the cell including, for example gene-expression data and the like. It should be noted that these applications are example applications only and one of skill will readily be able to apply the methods disclosed herein to any number of other different or variant applications.

Methods according to the present disclosure exploit conjugated binding agents.

In various embodiments, the invention provides a method of detecting a target non-nucleic acid molecule (e.g., a target protein) and a target nucleic acid molecule (e.g., a target mRNA) in a sample. The method generally comprises the steps of:
a) contacting a sample comprising a target non-nucleic acid molecule and a target nucleic acid molecule with a binding agent under conditions that permit binding between the binding agent and the target non-nucleic acid molecule in the sample, wherein the binding agent is covalently conjugated to an oligonucleotide that comprises a binding agent identification tag and a poly(A) sequence;
b) reverse transcribing the conjugated oligonucleotide and the target nucleic acid molecule in the sample simultaneously, thereby producing a conjugated oligonucleotide reverse transcription product and a target nucleic acid molecule reverse transcription product, wherein reverse transcription of the conjugated oligonucleotide is primed using an extension primer that comprises a poly(dT) sequence, wherein the length of the poly(dT) sequence is greater than or equal to the length of the poly(A) sequence in the conjugated oligonucleotide;
c) amplifying the conjugated oligonucleotide reverse transcription product and the target nucleic acid molecule reverse transcription product simultaneously, thereby producing a conjugated oligonucleotide amplicon and a target nucleic acid molecule amplicon; and
d) detecting: 1) the conjugated oligonucleotide amplicon, thereby detecting the target non-nucleic acid molecule in the sample; and 2) the target nucleic acid molecule amplicon, thereby detecting the target nucleic molecule in the sample.

A binding agent suitable for use in a method of this disclosure may possess a certain affinity and/or specificity profile. A useful binding agent with a particular "affinity" and/or "specificity" profile may bind (for example specifically bind) to a target.

The term "target" may include, for example, any molecule with an epitope, including but not limited to a protein (e.g., a glycoprotein, a cluster of differentiation (CD) marker), a carbohydrate (e.g., a sugar moiety on a protein), a lipid, or the like. The target may be any protein expressed by a cell - for example a cell membrane bound protein. In a particular embodiment, the target non-nucleic acid molecule is a protein.

As used herein, "target nucleic acid molecule" refers to a nucleic acid molecule comprising a sequence of contiguous nucleotides that is being analyzed (e.g., for expression level, for sequence information, or for the presence of a mutation). The target nucleic acid molecule can be, for example, DNA, cDNA or RNA (e.g., noncoding RNA or mRNA). In a particular embodiment, the target nucleic acid molecule is mRNA.

The binding agent may be an antibody. As used herein, unless otherwise indicated, "antibody" means an intact antibody or antigen-binding fragment of an antibody, including an intact antibody or antigen-binding fragment that has been modified or engineered. Examples of antibodies that have been modified or engineered are chimeric antibodies, humanized antibodies, multiparatopic antibodies (*e.*g., biparatopic antibodies), and multispecific antibodies (*e.g*., bispecific antibodies).

The binding agent may be a polyclonal or monoclonal antibody. The binding agent may comprise an antibody fragment - wherein the fragment is an epitope binding fragment or a fragment which retains the ability of the native (or complete) antibody to bind to its target. Examples of antigen-binding fragments include Fab, Fab', F(ab')₂, Fv, single chain antibodies (*e.g*., scFv), dAbs, minibodies and diabodies.

The binding agent may be a protein ligand, such as a ligand for a cell surface receptor.

The binding agent may be a carbohydrate-binding protein (e.g., a lectin).

The binding agent may be a lipid-binding protein.

The binding agent may be a small molecule or aptamer. For example, the binding agent may comprise an oligonucleotide with an affinity or specificity (i.e. an ability to bind) a target molecule (for example a protein, glycoprotein, CD marker or the like).

The binding agent may be conjugated to an oligonucleotide.

The oligonucleotide may comprise double or single stranded nucleic acid, or partially double stranded nucleic acid. In certain embodiments, the oligonucleotide is single stranded.

The oligonucleotide can have a length in the range of about 25 to about 110 nucleotides. In some embodiments, the oligonucleotide has a length of about 50 to about 80 nucleotides.

The binding agent may be conjugated, for example to an oligonucleotide, by any known method. In the case of binding agents which are antibodies, conjugation may require the provision of functionalised antibodies and oligonucleotides which may be generated via enzymatic reactions, chemical tagging and/or via the incorporation of nonnatural amino acids. For example, the binding agent (for example an antibody) may be conjugated using covalent strategies or non-covalent strategies, such as coupling via biotinstreptavidin or covalent conjugation using thiol-maleimide chemistry. The conjugation may be direct (i.e., without a linker) or through an intermediate structure, such as a linker oligonucleotide or other linker molecule, such as a cleavable linker. The conjugation may be reversible or irreversible. In particular embodiments, the binding agent is conjugated to an oligonucleotide via a covalent linkage.

The oligonucleotide conjugate may be blocked. In particular, the 3' end of the oligonucleotide may be blocked. Suitable blocking groups are known in the art. By way of example, the oligonucleotide conjugate may be blocked with some form of modified nucleotide. The purpose of the block may be to prevent a polymerase from extending a chain reaction beyond the modified base. In one example, the oligonucleotide conjugate may be blocked with a 3' ddNTP (for example a 3' ddGTP). Other methods of generating 3' blocks, including the use of inverted dT (which creates a 3'-3' linkage inhibiting degradation by 3' exonucleases and extension by DNA polymerases), or 3' phosphate groups, will be known to one of skill in this field.

The oligonucleotide conjugate may comprise a polyA tail. As will become apparent later, an advantage associated with the use of polyadenylated oligonucleotide conjugates is that the oligonucleotides may be able to participate in RNA sequencing processes. A polyA tail will typically contain about 14 to about 35 contiguous adenine bases, more typically about 25 to about 30 contiguous adenine bases.

The oligonucleotide conjugate may comprise a binding agent identification tag. As used herein, a "binding agent identification tag", or "binding agent barcode", refers to a sequence of nucleotides in an oligonucleotide that is conjugated to a binding agent, and which can be incorporated into extension products (e.g., reverse transcription products, amplicons) of the oligonucleotide. A binding agent identification tag can be used in sequencing applications to identify the particular binding agent to which the corresponding oligonucleotide is conjugated. For example, the binding agent identification tag can identify a particular antibody and, by correlation, the target protein that is bound by the antibody.

The oligonucleotide conjugate may comprise an affinity tag (e.g, a biotin molecule).

Thus disclosed herein are binding agents conjugated to oligonucleotides, binding agents conjugated to blocked oligonucleotides and binding agents conjugated to polyadenylated oligonucleotides.

The methods described herein may not require that the oligonucleotide conjugates are cleaved from the binding agent.

Further, the methods described herein may not require that the sample be immobilised to any particular substrate prior to contact between the sample and the conjugated binding agent and/or any subsequent nucleic acid amplification procedures.

The methods disclosed herein may use or exploit any of the disclosed conjugated binding agents.

Thus the conjugated binding agents (for example an antibody conjugated to an oligonucleotide) may be used to quantitate an amount of a protein in a sample.

The sample may comprise a biological sample, for example a tissue, fluid, secretion, biopsy or cell sample wherein the sample contains the protein or proteins to be quantified. As stated, the sample may comprise a cell. The sample may comprise a mixed population of cells. Prior to being subjected to a method of this disclosure a sample which comprises a mixed population of cells may be subject to a sorting procedure to yield a preparation comprising a single cell type. Single cell preparations may be obtained by FACS, droplet encapsulation or microfluidic based procedures. Other procedures for preparing single cells from multi-cell type populations will be known to those skilled in this field.

The protein to be quantitated may be a protein expressed by the cell. The protein may be a protein expressed at the cell surface - otherwise known as a membrane bound protein. The protein may be a glycoprotein for example a cell cluster of differentiation (CD) marker. One of skill in this field will be familiar with CD markers which include, for example CD3, CD4, CD8, CD45, CD16 and CD34 to name but a few. Cells can be characterised on the basis of the presence or absence of one or more such CD markers.

Thus, methods which use binding agents with an affinity for some cell membrane bound moiety (for example a CD marker or the like) can be used to determine whether a specific cell or cell type is present in a sample. Further, the methods may be used to count the number of cells in a sample or to determine how many cells there are of a particular type in a sample.

The methods described herein require that the conjugated binding agent be contacted with the sample to allow the conjugated binding agent to bind to its target. The binding agent may be contacted for a sufficient period of time to permit target binding and under conditions which are conducive to target binding.

For example, where a binding agent binds a particular cell CD marker, a conjugated form of that binding agent (conjugated according to this disclosure) may be contacted with a cell under conditions which permit binding between the binding agent and its CD marker target present on the cell surface.

Unbound conjugated binding agent may then be removed, for example by washing.

The methods may exploit two or more different types of binding agent.

The methods may exploit multiple (two or more different types of) binding agent(s). In one embodiment, one binding agent (or cohort of binding agents) may exhibit an affinity and/or specificity for one target and another binding agent (or cohort of binding agents) may exhibit an affinity and/or specificity for another target.

The various binding agents may comprise the same or different conjugates. For example binding agents with a common or shared affinity/specificity may be conjugated to the same (oligonucleotide) conjugate.

Thus methods which use multiple different types of conjugated binding agent (as described above) may be used in multiplex protein detection assays. For example a method which uses two types of binding agent - one with affinity and/or specificity for one protein target and another with affinity and/or specificity for another protein target may be used to quantitate two or more proteins in a sample. An application of a multiplex method of this type is counting the number of cell types in a sample - where conjugated binding agents with specificity for specific cell markers allow the user to determine how many cells of any given type are present in a sample.

In all cases, the quantification of a protein or proteins present in the sample may be achieved by determining the amount of each type of conjugate present after any necessary washing steps - the amount of conjugate present being directly proportional to the amount of bound binding agent.

Where the conjugate is a nucleic acid, for example an oligonucleotide, a nucleic acid amplification technique may be used to quantify the amount of conjugate present.

The nucleic acid amplification technique may be a PCR based technique. In some embodiments, the PCR based technique includes rapid amplification of cDNA ends (RACE). In some embodiments, the PCR based technique includes 5'-RACE. In some embodiments, the PCR based technique includes 3'-RACE.

For example, once the (oligonucleotide) conjugated binding agent has been allowed to bind to any of the target entity (protein) present in the sample and after any required washing procedure, the oligonucleotide conjugates may be amplified to generate an amplicon. For example, primers may be used to amplify the oligonucleotide conjugates. As used herein, the term "primer" refers to an oligonucleotide (e.g., single-stranded oligonucleotide), which is complementary to a template polynucleotide sequence and is capable of acting as a point for the initiation of synthesis of a primer extension product. A primer can be complementary to the sense strand of a polynucleotide sequence and act as a point of initiation for synthesis of a forward extension product. The primer can be complementary to the antisense strand of a polynucleotide sequence and act as a point of initiation for synthesis of a reverse extension product. The primer may occur naturally, as in a purified restriction digest, or be produced synthetically. The appropriate length of a primer depends on the intended use of the primer, but typically ranges from about 5 to about 200; from about 5 to about 100; from about 5 to about 75; from about 5 to about 50; from about 10 to about 35; from about 18 to about 22 nucleotides. A primer need not reflect the exact sequence of the template but must be sufficiently complementary to hybridize with a template for primer elongation to occur, i.e., the primer is sufficiently complementary to the template polynucleotide sequence such that the primer will anneal to the template under conditions that permit primer extension.

Primers can be free or immobilized (e.g., conjugated to a bead). In some embodiments, primers are immobilized on beads (e.g., a bead in a microwell or nanowell). The beads containing immobilized primers can include extension primers, gene specific primers for one or more genes, or a combination thereof.

The amount of generated amplicon and/or the rate and quantity of amplification can then be used as a means to determine or quantify the amount of the target entity (for example target protein) present in the sample. As such, the PCR based technique may be a quantitative PCR (QPCR) technique.

Where the method has used binding agents with different oligonucleotide conjugates, the identity and/or sequence of the resulting amplicons can be used as a further parameter by which to quantitate the amount of a specific protein or cell in a sample.

The nucleic acid amplification technique can be performed using any suitable nucleic acid polymerase enzyme or combination of nucleic acid polymerase enzymes. As used herein, the phrase "nucleic acid polymerase enzyme" refers to an enzyme (e.g., naturally-occurring, recombinant, synthetic) that catalyzes the template-dependent polymerization of nucleoside triphosphates to form primer extension products that are complementary to one of the nucleic acid strands of the template nucleic acid sequence. Numerous nucleic acid polymerases are known in the art and are commercially available. Nucleic acid polymerases that are thermostable, e.g., they retain function after being subjected to temperatures sufficient to denature annealed strands of complementary nucleic acids, are particularly useful for the methods of the present invention.

The nucleic acid amplification technique may be used to introduce specific tags, sequences or barcodes into an amplicon. For example, a nucleic acid amplification technique may be used to introduce specific tags, sequences or barcodes into an amplicon, such as a binding agent identification tag, a cell identification tag, a unique molecular identifier (UMI) sequence, or a primer binding site (e.g., for binding of sequencing primers).

As used herein, a "cell identification tag", or "cell identification barcode", refers to a sequence of nucleotides that can be incorporated into extension products (e.g., amplicons) and used in sequencing applications to identify the particular cell (e.g., a single cell) or cell type in which the extension product(s) was generated. In the methods described herein, a cell identification tag can be present in an oligonucleotide that is conjugated to a binding agent, or can be included in a primer (e.g., an extension primer, such as an oligo(dT) primer, or an amplification primer) for introduction into an extension product (e.g., a RT product, an amplicon). A cell identification tag can be incorporated into an extension product by a suitable nucleic acid polymerase, such as a reverse transcriptase enzyme or a DNA polymerase enzyme.

"Unique molecular identifiers" or "UMIs", which are also called "Random Molecular Tags (RMTs) ", are sequences of nucleotides that are used to tag a nucleic acid molecule (e.g., prior to amplification) and aid in the identification of duplicates. UMIs are generally random sequences and typically range in size from about 4 to about 20 nucleotides in length. Examples of UMIs are known in the art. Such UMIs are useful in the present invention.

In some embodiments of the methods described herein, a conjugated oligonucleotide is reverse transcribed by a reverse transcriptase enzyme to yield a reverse transcription product, or RT product, referred to herein as a conjugated oligonucleotide reverse transcription product. The conjugated oligonucleotide reverse transcription product can be single stranded (e.g., when the oligonucleotide comprises a blocking group and/or is conjugated to the binding agent at its 3' end) or can be fully or partially double stranded (e.g., when the oligonucleotide does not include a blocking group and is conjugated to the binding agent at its 5' end). In particular embodiments, reverse transcription of a conjugated oligonucleotide comprises extending the 3' end of the conjugated oligonucleotide that has annealed to an extension primer with a reverse transcriptase enzyme to thereby produce a double stranded conjugated oligonucleotide reverse transcription product.

Reverse transcription of a conjugated oligonucleotide and/or a target nucleic acid molecule (e.g., target mRNA) can be primed with an extension oligonucleotide, or extension primer, that is complementary to a nucleotide sequence in the conjugated oligonucleotide. In the case of a conjugated oligonucleotide that comprises a poly(A) tail, the extension primer can comprise a complementary poly(dT) sequence (e.g., poly(T) tail at its 3' end). The poly(dT) sequence in the extension primer has the same length as, or longer, than a corresponding poly(A) sequence in a conjugated oligonucleotide.

The extension primer can comprise one or more features selected from a cell identification tag, a unique molecular identifier (UMI) sequence, a poly (dT) sequence, and a non-dT sequence that is complementary to a nucleotide sequence in the conjugated oligonucleotide, or a combination of any of the foregoing. In some embodiments, the extension primer comprises a template switching oligonucleotide.

Reverse transcription of a conjugated oligonucleotide and one or more target nucleic acid molecules (e.g. cellular mRNA molecules) is performed simultaneously, thereby producing a conjugated oligonucleotide reverse transcription product and a target nucleic acid molecule reverse transcription product. For example, reverse transcription of the conjugated oligonucleotide and one or more target nucleic acid molecules can be primed with a single extension primer that is capable of hybridizing to the conjugated oligonucleotide and the one or more target nucleic acid molecules (e.g., an extension primer comprising an olig(dT) sequence), or by priming with two or more different extension primers (e.g., a non-dT template switch oligonucleotide primer for the conjugated oligonucleotide and an oligo(dT) primer for cellular mRNA).

In some embodiments, reverse transcription of the one or more target nucleic acid molecules (e.g., cellular mRNA molecules) is part of a cDNA library generation process. The cDNA library can be a general cDNA library (e.g., a cDNA library for total mRNA from a cell) or a targeted cDNA library.

Thus, in some embodiments, both target and non-target nucleic acid molecules in a sample are reverse transcribed, thereby producing target nucleic acid molecule reverse transcription products and non-target nucleic acid molecule reverse transcription products. When both target and non-target nucleic acid molecule reverse transcription products are generated, the target nucleic acid molecule reverse transcription product can be selectively amplified. Alternatively, the target nucleic acid molecule reverse transcription product and the non-target nucleic acid molecule reverse transcription products can both be subsequently amplified in the same reaction.

When reverse transcription of a conjugated oligonucleotide and one or more target nucleic acid molecules (e.g. cellular mRNA molecules) is performed simultaneously (e.g., in the same reaction), the resulting conjugated oligonucleotide reverse transcription product and target nucleic acid molecule reverse transcription product can each be amplified to produce a conjugated oligonucleotide amplicon and a target nucleic acid molecule amplicon. The conjugated oligonucleotide amplicon and target nucleic acid molecule amplicon can be separated, for example, prior to a second round of amplification and/or prior to detecting the amplicons. Separation of the conjugated oligonucleotide amplicon and target nucleic acid molecule amplicon can be based on amplicon size or binding to labels, such as affinity tags. In some embodiments, separation of the conjugated oligonucleotide amplicon and target nucleic acid molecule amplicon is not required prior to further amplification and/or detection of the amplicons.

In some cases, the oligonucleotide conjugate may be amplified in order to yield DNA for sequencing.

In some embodiments, the conjugated oligonucleotide reverse transcription product and target nucleic acid molecule reverse transcription product can be amplified in order to yield DNA for sequencing.

During amplification, binding sites for sequencing primers can be added to the amplicons being generated. For example, binding sites for sequencing primers can be added using transposons in accordance with standard techniques known to those of skill in the art. Alternatively, or in addition, binding sites for sequencing primers can added by fragmenting nucleic acid molecules in the amplicons and ligating the binding sites to the fragments. As would be understood by those of skill in the art, binding sites for sequencing primers can also be added using primers, for example, by a primer extension reaction or a nucleic acid amplification reaction.

As stated, where the sample comprises cells and where the conjugate of the binding agent is an oligonucleotide, once any required washing step (to remove unbound conjugated binding agent) is complete the sample may be subject to a cell sorting procedure. The cells may be sorted directly into medium containing reagents which permit nucleic acid amplification. Those reagents may be reagents for PCR. Reagents for PCR (or for any nucleic acid amplification technique) may include dinucleotides (dNTPs), buffers, polymerase enzymes and probes or primers. For example if the PCR reagents contain primers complementary to a sequence of the oligonucleotide conjugate, under the correct cycling conditions, a reaction can proceed and there will be DNA amplification.

As stated:
(i) after the contacting step, unbound binding agent may be removed. This may be achieved via any suitable technique - including, for example, affinity chromatography or the like. However, unbound binding agent may be removed by washing and the cells may be washed any number of times in order to remove unbound conjugated binding agent.
(ii) the cells may be sorted. For example after the contacting and/or washing step, the cells may be sorted. The cells may be sorted by any suitable cell sorting technique including, for example FACS, droplet encapsulation and/or microfluidics. Sorting techniques may be used to obtain populations of single cells. For example where a sample contains multiple different types of cells, a sorting technique may be used to split the sample into smaller populations containing fewer, or even single, cell types. The cells may be sorted directly into medium comprising reagents for a nucleic acid amplification procedure.

Thus this disclosures provide methods which use oligonucleotide conjugated binding agents to quantitate non-DNA molecules (for example proteins) - the binding event (between the binding agent and a non-DNA target molecule) essentially serving to convert the non-DNA molecule into a specific DNA molecule (i.e. the oligonucleotide conjugate).

As stated, a sorting procedure for use in or with any of the methods described herein may comprise droplet based methods. Additionally or alternatively a droplet based sorting technique may be combined with a nucleic acid amplification procedure. For example, a method of this disclosure may exploit a technique such as fluorescent PCR based droplet sorting. In techniques of this sort a stained cell (for example a cell labelled with a conjugated binding agent disclosed herein) may be sorted or compartmentalised into a droplet and within that droplet a nucleic acid amplification process occurs - this may take the form of a fluorescent PCR. Within the droplet a successful or completed PCR reaction generates a fluorescent amplicon from the oligonucleotide conjugate of the binding agent and the droplets can be sorted on the basis of this signal (or lack thereof). Further, these droplet based methods could include other nucleic acid amplification process which amplify additional oligonucleotide conjugates within the droplet. Additionally, or alternatively, the amplicons can be sequences.

Other processes may include techniques in which droplet encapsulated cells can be sorted by genotyping PCR method with the protein content being measured, determined or quantified by sequencing.

A further aspect of the methods described herein is that the nucleic acid amplification techniques (for example PCR based techniques) which are used to amplify the oligonucleotide conjugates can also (or simultaneously) be used to generate information relating to some nucleic acid based aspect of the same sample.

By way of example, the methods may be used to further generate information relating to cellular nucleic acids including for example information relating to genomic sequences, genes, RNA, gene expression, mitochondrial nucleic acid, plasmid sequences, viral sequences and the like.

When extracting data from samples which contain multiple different sources of protein (for example samples which comprise a population of different cell types), it is necessary to normalize the input amounts. This may be done by testing the DNA or RNA input from the sample to normalize the source or cell number. For example, the methods described herein may use information obtained from the nucleic acid content of the sample (for example any intracellular nucleic acid (RNA) or genomic DNA) to normalise any proteomic information obtained from the binding of the conjugated binding agent and subsequent amplification of the oligonucleotide conjugate.

Thus the oligonucleotide of the conjugated binding agent may be co-amplified together with some other nucleic acid present in the sample. That other nucleic acid may be an intracellular nucleic acid for example a part of a genome, a plasmid nucleic acid, an RNA and/or mitochondrial DNA (mtDNA).

Thus where the sample comprises a cell, for example a mammalian cell, a PCR based technique (for example QPCR) may be used to amplify the oligonucleotide component of the conjugated binding agent (using probes or primers for the oligonucleotide conjugates) and a mitochondrial gene (for example MtND1 - using another set of probes or primers to amplify the MtND1 gene).

The different amplicons may be labelled differently - for example the nucleobases, probes and/or primers for each different amplification reaction may be labelled with different (fluorescent) dyes or tags. For example fluorescein (FAM) and/or VIC type dyes may be used. One of skill will be aware of a range of other dyes that may be used to label probes and/or primers - these may include TET, JOE, HEX, NED, PET, ROX, TAMRA, TET and/or Texas Red type dyes.

Once the amplification step of the method is complete (which amplification step may co-amplify both the oligonucleotide conjugate of the conjugated binding agent and some other (for example intracellular) nucleic acid, average threshold cycle (Ct) data for each amplification reaction may be obtained.

Ct data for the amplification reaction proceeding from the oligonucleotide conjugate of the conjugated binding agent may be compared to Ct data for the amplification reaction proceeding from the other (for example intracellular (perhaps mtDNA)) nucleic acid in order to yield a normalisation factor between repeat experiments (or assays) or different samples.

The normalisation factor may be used to normalise the cell numbers between samples and/or repeat experiments.

The methods described herein may comprise or further comprise a reverse transcriptase (RT) based protocol for the generation of cDNA from RNA. For example, the methods may subject samples to a RT process that generates cDNA from any RNA content within the sample. The sample may comprise a cell or cells and thus the RT process may be used to obtain cDNA which can itself be used to determine levels of gene expression within a cell, T-cell receptor and/or antibody expression and/or sequence data.

Where one is intending to use an RT process, the oligonucleotide conjugated to the binding agent may be a blocked oligonucleotide as described above.

The RT process may be restricted to a few or low number of cycles. For example, the RT process may be restricted to 3, 6, 9, 12, 15, 18, 21 or more cycles.

The generated cDNA and the (blocked) oligonucleotide conjugates of the binding agent may then be subjected to a further nucleic acid (DNA or cDNA) amplification process. This may be a tagmentation reaction and/or some standard QPCR process.

Thus the methods of this invention provide a means by which a single readout (DNA sequencing or the like) may be used to provide information about proteins (for example cell membrane proteins) and RNA (or gene expression) in a sample.

In all cases, the DNA amplification part of the method may take the form of a co-amplification process in which both the nucleic acid conjugated to the binding agent, any cDNA generated by a RT process and/or any other intracellular nucleic acid as described herein, are amplified together and in the same process.

Thus a method of quantifying the number of cells expressing a particular protein in the sample may comprise:
providing a sample comprising a cell;
contacting the cells with a conjugated binding agent of this disclosure, wherein the binding agent exhibits affinity/specificity for a protein target and the binding agent and sample are contacted under conditions which permit binding between the agent and any of its protein target in the sample;
subjecting the sample to a reverse transcription process to generate cDNA from the cell;
subjecting the sample to a DNA amplification process to generate amplicons from both the oligonucleotide conjugated to the binding agent and the cDNA from the cell;
detecting, sequencing or quantifying the amplified nucleic acid in order to quantify the number of cells expressing the protein target in the sample.

In a method of this type:
(i) the binding agent may be conjugated to a blocked oligonucleotide; and
(ii) the DNA amplification process may be a QPCR or multiplex QPCR process.

The RT process may use polydT primers and/or random primers.

Specific methods for measuring RNA and proteins in the same sample (of cells) with the same readout (or perhaps just a sequencing readout) and which fall within the scope of this disclosure may include, for example those detailed in (non-limiting) examples 1-5 below:
1. A sample comprising cells is contacted (or stained) with a DNA conjugated binding agent (for example an antibody) in which the 3' end of DNA has been blocked (as described elsewhere.

The DNA conjugated binding agent may exhibit affinity and/or specificity for a protein expressed by a cell (note - a cell expressing that protein may or may not be present in the sample of cells provided) under conditions which permit binding between the conjugated binding agent and any of its protein target in the sample. Optionally the sample may be subject to a washing procedure in order to remove unbound binding agent.

Thereafter, the (washed) sample may be subject to a RT process in order to generate cDNA from the cells of the sample. This may be achieved using, for example a clontech SMARTer kit.

The generated cDNA may then be selectively amplified to generate a RNAseq library. Techniques such as Illumina's tagmentation technology, may be used.

Both the RNAseq library AND the DNA conjugated to the binding agent may then be co-amplified. In this process it may be possible to add a sample barcode and prepare the library for sequencing. In processes such as this it may be important that the DNA conjugated to the antibody is single stranded as this will prevent it from reacting with the process (for example the tagmentation reaction) used to generate the RNAseq library.

2. Other RNAseq library including those used in droplets where sample barcode is added at the cDNA generation step. In other embodiments, the oligonucleotide conjugate of conjugated binding agent may be a modified DNA modified to include a polyA tail. Oligo synthesis techniques and/or polyA polymerase may be used.

As with other methods described herein a sample containing cells may be contacted (or stained) with the conjugated binding agent (for example an antibody) under conditions which permit binding between the binding agent and any cell which expresses the protein target of the binding agent. As before, it should be understood that a cell expressing the protein target of the binding agent may or may not be present in the sample of cells provided. Optionally the sample may be subject to a washing procedure in order to remove unbound binding agent.

Thereafter, the (washed) sample may be subject to a RT process with oligo-dT which contains a sample barcode. This will generate first and second strand cDNA (also creates a double stranded DNA conjugated antibody). The DNA may then be sheared and ligated to sequencing adaptors so as to generate a library for sequencing. A key feature of this method is that the DNA which is conjugated to the binding agent (antibody) is treated just like the RNA.

3. Other methods may involve the amplification of specific loci within the cDNA (such as gene fusion junctions, T-cell receptor loci and the like). These may be sequenced in a manner similar to the gDNA amplification procedures described above

4. A variant method may but include a DNA based PCR step that adds (for example) illumina adaptors to the genomic DNA amplicon.

5. Other methods may involve RNA readout using golden gate assay techniques, molecular inversion probes or proximity ligation (e.g., temo-o-seq/ligation chain reaction). In this embodiment, probes hybridizing to RNA or cDNA can have the same (Illumina) handle sequences as the conjugated binding agent (for example antibody-oligonucleotide conjugates) or different handle sequences. The use of different handle sequences may be preferred so that two different PCRs can be conducted, allowing for control over the amount of RNA vs protein signal that gets sequenced.

The present disclosure provides methods in which a single (or the same) cell can be analysed with the conjugated (for example DNA tagged) binding agents (for example antibodies) described herein and some form of nucleic acid (DNA) amplification process. The nucleic acid amplification (including genomic DNA amplification) may be used as a means to achieve the relative normalization of protein counts, cell counting or genotyping.

In some embodiments the amount or level of genomic DNA amplification is limited. For example the methods may exploit genomic DNA amplification which is restricted or limited by the use of limited cycle amplification. Any limited cycle genomic DNA amplification may exploit primers which comprise 'handles' for sequencing adaptors. The principle is that the level of genomic amplification must be sufficient for it not to be swamped or masked by any amplification process which proceeds from the oligonucleotide of the conjugated binding agent. Indeed the level of genomic DNA amplification and the level of conjugated oligonucleotide amplification must be balanced so that neither product is swamped or masked.

To achieve this:
(a) the level of genomic DNA amplification may be limited by the use of limited cycle PCR. Thereafter, sequence adaptors for additional PCR of both the genomic DNA amplicons and the oligonucleotides of the conjugated binding agents may be used;
(b) the level of genomic DNA amplification may be limited by the use of limited cycle PCR followed by primer removal either by primer digest or PCR cleanup technology (for example ampure beads and the like). Following this additional PCR with sequence adaptors may be performed;
(c) the level of genomic DNA amplification is limited by the use of limited cycle PCR, then the reaction is diluted into a new PCR reaction containing sequence adaptors; and/or
(d) the level of genomic DNA amplification is limited by the use of limited cycles with primers containing binding sites for a first sequence adaptor 1, which is different from the binding sites for the oligonucleotides of the conjugated binding agents. Thereafter primers for a second sequence adaptor which binds to the oligonucleotide of the conjugated binding agent are added.

The methods of the invention can be for example performed using kits, the kits comprising conjugated binding agents as described herein and instructions for use in any of the methods of this disclosure. For example the kits may contain antibodies with specificity for proteins expressed by cells, which antibodies are conjugated to oligonucleotides.

The kit may be for counting or quantifying cells in which case the kits may at least comprise a conjugated binding agent with affinity and/or specificity for a protein expressed by the cell to be counted.

As explained in more detail above, a kit of this type may be used to count or quantify a particular cell type within a sample.

Thus the kits may comprise oligonucleotide conjugated antibodies, wherein the antibody component of the conjugate exhibits a specificity or affinity for a protein expressed by a cell.

The kits may further comprise reagents for a nucleic acid amplification process. For example the kits may comprise reagents for PCR type reactions including (labelled) primers, (labelled) deoxyribonucleotide triphosphates (dNTPs), buffers, enzymes (for example polymerase enzymes) and the like.

The kit may comprise or further comprise reagents for reverse transcriptase reactions. Such reagents may include for example reverse transcriptase enzymes.

The kits may contain receptacles in which the methods and its various stages may be conducted.

In certain embodiments, the kit may be used for detecting a target protein and a target nucleic acid molecule in a sample. The kit generally comprises a binding agent that is conjugated to an oligonucleotide, and one or more DNA amplification primers. The binding agent in the kit is covalently conjugated to an oligonucleotide.

In certain embodiments, a kit can comprise multiple binding agents having affinity and/or specificity for two or more different targets (e.g., two or more different target proteins).

The conjugated oligonucleotide further comprises a binding agent identification tag.

In some embodiments, the binding agent is an antibody, such as a monoclonal antibody.

In some embodiments, the conjugated oligonucleotide is a DNA molecule. In certain embodiments, the DNA molecule is single stranded.

In some embodiments, the conjugated oligonucleotide comprises a blocking group that prevents extension of the primer by a polymerase. In particular embodiments, the blocking group is a ddNTP or an inverted dTTP. In certain embodiments, the blocking group is at the 3' end of the conjugated oligonucleotide.

The kits can further comprise one or more additional components, such a reagents for performing a reverse transcription reaction (e.g., a reverse transcriptase (RT) enzyme, an oligo dT primer, a reverse transcription buffer, deoxynucleotides (dNTPs), or a combination thereof) and/or reagents for performing a nucleic acid amplification reaction (e.g., a DNA polymerase, an amplification buffer, deoxynucleotides (dNTPs), or a combination thereof).

In certain embodiments, the kit can also comprise one or more sequencing primers.

The methods of the invention can be for example performed using a binding agent conjugated to blocked oligonucleotide.

In some embodiments, the binding agent comprises antibody or an epitope binding fragment thereof. In certain embodiments, the antibody is a monoclonal antibody.

In some embodiments, the binding agent comprises a small molecule or aptamer.

In some embodiments, the binding agent has affinity and/or specificity for, or an ability to bind to, a protein. In particular embodiments, the protein is a protein expressed by a cell. In some embodiments, the protein is a cell membrane bound protein. In certain embodiments, the protein is a cluster of differentiation (CD) glycoprotein.

In some embodiments, the binding agent is conjugated to a polyadenylated oligonucleotide. In certain embodiments, the oligonucleotide is 3' blocked. In particular embodiments, the oligonucleotide is blocked by the addition of a modified nucleotide. In some embodiments, the oligonucleotide is blocked by the addition of a 3' ddNTP or an inverted dTTP. In certain embodiments, the oligonucleotide is blocked by the addition of a 3' ddGTP.

In various embodiments, the method of the invention can be used for measuring or quantifying protein and/or nucleic acid, or the number of cells (e.g., number of cells expressing a target protein), in a sample. In certain embodiments, the method comprises the steps of contacting a sample with a binding agent conjugated to an oligonucleotide and/or a conjugated binding agent as described herein. The binding agent exhibits affinity/specificity for a protein target, and the binding agent and sample are contacted under conditions which permit binding between the binding agent and any of its protein target in the sample. The method further comprises amplifying the oligonucleotide conjugate amplifying the oligonucleotide conjugate via a nucleic acid amplification technique; and detecting, sequencing or quantifying the amplified nucleic acid in order to quantify the amount of the protein, nucleic acid or number of cells (e.g., number of cells expressing a target protein), or any combination thereof, in the sample.

In some embodiments, the method includes sequencing the amplicon(s), wherein the sequence information is used to quantify the protein and/or nucleic acid present in the sample.

In certain embodiments, the method further comprises amplifying the oligonucleotide conjugate and any nucleic acid present in the sample by amplifying the oligonucleotide conjugate via a nucleic acid amplification technique quantitative PCR (qPCR). The qPCR data can be used to quantify the protein and/or nucleic acid present in the sample.

In some embodiments, the method is applied to a single sample and the protein and nucleic acid are quantified in the same sample.

In some embodiments, the binding agent is an antibody or an epitope binding fragment thereof.

In some embodiments, the binding agent is a small molecule or an epitope binding fragment thereof.

In some embodiments, the binding agent exhibits affinity and/or specificity for a glycoprotein or CD marker expressed by a cell.

In some embodiments, the sample comprises a single cell type, a population of cells or a mixed population of cells.

In some embodiments, the method further comprises removing unbound binding agent (e.g., by washing) once the sample has been contacted with the binding agent.

In some embodiments, after the contacting step and/or any optional unbound binding agent removal or washing step, the cells are sorted. In certain embodiments, the cells are sorted by FACS, droplet encapsulation or microfluidic based processes.

In some embodiments, the nucleic acid amplification technique or process is a polymerase chain reaction (PCR) based process.

In some embodiments, the cells are sorted directly into a medium containing one or more reagents for PCR.

In some embodiments, the nucleic acid amplification technique is further used to amplify a nucleic acid present in the sample.

In some embodiments, the nucleic acid amplification technique is a PCR based technique. In certain embodiments, the nucleic acid amplification technique is QPCR.

In some embodiments, the nucleic acid is a cellular nucleic acid.

In some embodiments, the nucleic acid comprises a cellular nucleic acid, genomic DNA, mitochondrial DNA, RNA, plasmid DNA, bacterial nucleic acid and/or viral nucleic acid.

In some embodiments, the amplification of nucleic acid present in the sample occurs first and before amplification of the oligonucleotide conjugated to the binding agent.

In some embodiments, amplification of nucleic acid present in the sample is limited. In certain embodiments, amplification of nucleic acid present in the sample is limited by limited cycle PCR. In some embodiments, the limited cycle PCR is followed by removal of primers specific for a nucleic acid present in the sample and/or digestion, denaturation or destruction of the same. In particular embodiments, the limited cycle PCR to amplify a nucleic acid of the sample, the primers specific for that nucleic acid are removed by a PCR clean-up process. In certain embodiments, the limited cycle PCR is followed by dilution of the PCR reagents into new reagents for the amplification of at least the oligonucleotide conjugated to the binding agent.

In some embodiments, the method comprises or further comprises a reverse transcriptase (RT) process. In certain embodiments, the RT process is used to generate cDNA from RNA present in or of, the sample. In particular embodiments, the RNA may be cellular RNA. In some embodiments, the sample comprises a cell or cells and the RT process is used to obtain cDNA from the RNA content of the cell or cells. In certain embodiments, the RT process comprises a restricted, reduced or limited number of cycles. In particular embodiments, the RT process comprises 3, 6, 9, 12, 15, 18, 21 or more cycles. In some embodiments, the RT process is conducted before the nucleic acid amplification process.

In some embodiments, information obtained from the nucleic acid amplification and/or RT process is used to normalise any protein or cell quantification data.

In various embodiments, the method of the invention can be used for quantifying a protein in a sample, comprising contacting the sample with a binding agent conjugated to an oligonucleotide, wherein the binding agent exhibits affinity/specificity for a protein target and the binding agent and sample are contacted under conditions which permit binding between the agent and any of its protein target in the sample; subjecting the sample to a reverse transcription process to generate cDNA from any RNA present in the sample; subjecting the sample to a DNA amplification process to generate amplicons from both the oligonucleotide conjugated to the binding agent and the cDNA from the cell; and detecting, sequencing or quantifying the amplified nucleic acid in order to quantify the number of cells expressing the protein target in the sample.

In various embodiments, the method of the invention can be used for quantifying the number of cells expressing a particular protein in the sample, comprising providing a sample comprising a cell; contacting the cells with a binding agent conjugated to an oligonucleotide and/or a conjugated binding agent described herein, wherein the binding agent exhibits affinity/specificity for a protein target and the binding agent and sample are contacted under conditions which permit binding between the agent and any of its protein target in the sample; subjecting the sample to a reverse transcription process to generate cDNA from the cell; subjecting the sample to a DNA amplification process to generate amplicons from both the oligonucleotide conjugated to the binding agent and the cDNA from the cell; and detecting, sequencing or quantifying the amplified nucleic acid in order to quantify the number of cells expressing the protein target in the sample. In some embodiments, the binding agent is conjugated to a blocked oligonucleotide. In some embodiments, the DNA amplification process is a PCR based process and/or a QPCR based process. In some embodiments, an amplicon product of the nucleic acid amplification process is sequenced. In certain embodiments, the sequence information is used or further used to obtain information about the genotype of a cell in the sample and/or the expression of one or more genes.

### Example 1

### Quantitating non-DNA molecules

In this example, non-DNA molecules are quantitated by converting them into specific DNA molecules, which are then amplified and sequenced via high throughput DNA sequencing. The example shows how the methods may be applied to single cell based protein measurements, but the same or similar methods may be applied to multi-cell measurements.

Antibodies (Biolegend) were labelled with different DNA sequences depending on the final readout (qPCR or sequencing).

Antibody conjugation was performed by reducing the antibodies in TCEP and reacting them with maleamide labelled oligos (IDT - originated as 5' amine oligos which were converted to maleamide via NHS ester formation with maleamide).

Peripheral blood mononuclear cells (PBMCs) were purified using standard methods and were stained in the following way: ~1million cells were added to wells of a round bottom 96 well plate and were blocked in buffer containing 10% rabbit serum, 2%BSA,1mg/ml salmon sperm DNA, 0.2mg/ml cysteine in TBS. All steps were performed on ice. After blocking for 10 minutes, cells were spun down for 3min at 500g and supernatant removed.

Antibody was added at 1ng/µl in blocking solution and incubated for 30 minutes.

Cells were spun down and supernatant removed followed by washing 1X 5min in blocking buffer and 3X2 min in TBS.

Cells were re-suspended in TBS and sorted using the Sy3200 cell sorter to collect either CD3 positive cells or live cells (via DAPI exclusion).

One cell was added to each well of a 96 well plate which contained 20ul of PCR reagents for further processing of the DNA.

DNA sequences used:
For Taqman: assays were developed using sequences and taqman kits provided by IDT. The antibody conjugates were as follows: anti-CD4 was conjugated to gTcaIGCCGTAGCAGTTGAGGAAGTTGGTAGCGGTGGATGGCGAGAGTACACTTGA GACAAGCACCAGAAAGATCA (SEQ ID NO:1) and anti-CD8 was conjugated to gatcgtIGCAAACAAAGTCTGGCCTGTATCCTAGCTTCGAGAGGTCCTTTTCACCAGC ACTGTACTGCTTGACCAAGGAA (SEQ ID NO:2).

DNAs were amplified in the taqman assay using the corresponding IDT kits for targeting either the B2M gene (CD4) or the HPRT1 gene (CD8).

Reactions were performed on a Thermo-quantstudio 6 using Vic and FAM as the dyes. PCRs were 95°C for 3min, followed by 40 cycles of 95°C for 10 seconds, 62°C for 30 seconds using BioRad 2X qPCR probe mastermix. Analysis was performed using default settings.

For DNA sequencing assays, anti CD4 and anti-CD19 were used and were conjugated to oligos: CD4-GACTGGAGTTCAGACGTGTGCTCTTCCGATCTCGTCCATTNNNNNNNNNNNNAG ATCGGAAGAGCGTCGTGTAGGGAGATC (SEQ ID NO:3) and CD19-GACTGGAGTTCAGACGTGTGCTCTTCCGATCTGGAATGTCCATCCAAGNNNNNN NNNNNNNNNAGATCGGAAGAGCGTCGTGTAGGGAGATCTTTTT (SEQ ID NO:4).

DNAs were PCR amplified using 1X NEB Taq mastermix with the following primers:
AATGATACGGCGACCACCGAGATCTACACTCTTTCCCTACACGACGCTCTTCCGA TCT (SEQ ID NO:5) and
CAAGCAGAAGACGGCATACGAGATXXXXXXXXGTGACTGGAGTTCAGACGTGT G (SEQ ID NO:6) where X is an Illumina index sequence which is different for each well of a 96 well plate. PCRs were performed at 95°C for 3min, followed by 25 cycles of 95°C for 10 seconds, 62°C for 30 seconds, 72°C for 15 seconds.

Following PCR, 5ul of reaction was removed from each well and combined into a single tube. Ampure bead cleanup was run on the combined sample using a 2:1 bead ratio and following manufacturer's instructions.

DNA sequencing was performed on an Illumina Miseq following standard protocols. Data was analyzed in the following way:

Quality trimming of raw fastq files was performed using Trimmomatic. Adaptor sequences were removed using a custom script based on Biopieces. Duplicate sequences with identical barcode and UMI sequences were collapsed and the number of reads with unique UMIs were tabulated. A custom perl script was then used to sort the collapsed reads into the CD4 and CD19 bins where reads were normalized by the total number of reads obtained for each sample.

An outline process is shown in FIG. 1.

### Results

### Single cell protein measurements

For single cell measurements, peripheral blood mononuclear cells (PBMCs) were used -these being a well characterized cell type that allows us to make measurements of known proteins. Initial studies were focused on CD3 and CD4 positive T-cells.

Thawed PBMCs were co-stained for both CD3 and CD4 using commercially available, fluorescently labelled antibodies. FIG. 2 shows the resulting FACS plot in which 56.6% of the CD3 positive cells were found to be CD4 positive for our particular PBMC isolation.

PBMCs were then stained with a fluorescent anti-CD3 antibody and a DNA labelled anti-CD4 antibody. This particular DNA label is specific for a taqman based quantitative PCR assay.

Cells were sorted into individual wells of a 384 well plate containing PCR reagents. Following PCR, cells could clearly be separated into positive and negative populations, where CD4 positive cells have lower Ct values (that is, the DNA amplifies faster, equating to more original starting DNA molecules) while the higher Ct values represent cells that are negative for CD4.

In the assay shown in FIG. 2, 58% of the cells measured were positive for CD4, showing a strong correlation to the FACS data.

The number of protein molecules being measured per cell may be noted; the use of quantitative PCR to make the measurements, generates a standard curve of DNA inputs to define the number of starting DNA molecules found on each single cell. While small variations in buffer conditions across assays may play a role, on average the positive cells contain >1000 DNA tags per cell while the negative cells signal represents <10 molecules of background signal.

Although initial data consisted of two protein measurements, one with DNA and one with fluorescence, there is repeatable data measuring three proteins per sample. For these experiments, a fluorescent anti-CD3 antibody which was used to sort for CD3 positive cells; the cells were then co-stained with DNA tagged anti-CD4 and CD8. The anti CD4 and CD8 antibodies were labelled with unique DNA tags that could be independently read out by qPCR for each cell. CD4 and CD8 are mutually exclusive in expression and across 192 cells measured, only two examples of double positive cells were detected, which is expected based on the known frequency of such events. The methods and techniques described herein clearly recapitulates the biology of the system and with the extremely low background signal - most cells positive for one antibody have zero PCR signal from the other (see FIG. 3).

### Multiplex protein detection in single cells using high throughput sequencing

Cells were labelled with anti-CD19 and anti-CD4 - this labelled B-cells and T-cells respectively. Individual cells were sorted into wells and DNA was PCR amplified for sequencing on an Illumina Miseq. Results (see FIG. 4) show no overlap between the two cell populations and little to no background signal. Counts were normalized by total sequencing reads and only unique DNA molecules were counted.

### Using gDNA to normalize cell number

PBMCS were stained as described above with a fluorescent anti-CD3 plus DNA tagged anti-CD4 and CD8. Cells were sorted into wells at either 10 cells/well or 50 cells/well. qPCR was performed using taqman probes for the antibody conjugates described above both labelled with FAM and co-amplified with probes against mitochondrial gene MtND1 labelled with VIC.

Following co-amplification, cells were averaged for VIC and FAM Cts.

Cellular DNA can be used to normalize protein counts and when mtDNA is used with at least 10 cells, it is possible to generate aa normalizing factor for use between samples.

Table 1 shows the results of an experiment in which cells were stained with fluorescent anti-CD3 and DNA tagged anti-CD4. Cells were sorted into either 50 cells/ well of CD3 positive, 100 cells/well of CD3 positive or 50 cells cd3 positive + 50 cells CD3 negative (by definition these would also be CD4 negative).

**Table 1**

| **Cells** | **Avg Ct (CD4)** | **Fold Change compared to 50 cells** | **Avg Ct (Mtnd1)** | **Fold Change compared to 50 cells** | **Avg CD4 signal per cell** |
|---|---|---|---|---|---|
| 50 | 20.62 | | 33.18 | | 1 |
| 100 | 19.47 | 2.22 | 32.12 | 2.09 | 1.06 |
| 50+50 | 20.32 | 1.23 | 32.46 | 1.73 | 0.71 |

### 3' blocking of antibody conjugated oligo

An anti-CD4 antibody was conjugated to either a standard oligonucleotide or an oligonucleotide that had been blocked with a 3' ddGTP via terminal transferase. PBMCs were stained as above with either CD4 (conjugated to a normal oligonucleotide) or CD4B (conjugated to a blocked oligonucleotide). 600 CD3 positive cells were sorted into 10µl of Clontech Smart-Seq v4 ultralow input RNA kit for sequencing. cDNA was generated following manufacturer's protocol and was amplified over 9 cycles. 150pg of each cDNA was used as input for illumina tagmentation using Nextera XT following the Illumina protocol.

5µl of the final tagmented product or equivalent mass of non-tagmented cDNA was used as template in a taqman reaction (described above) for a total volume of 20µl.

FIG. 5 shows that in qPCR the blocked oligo was not impacted by the tagmentation reaction, whereas the unblocked oligo lost ~50% of its signal (2 fold decrease in signal).

| Sample Name | CT |
|---|---|
| 4 | 30.838 |
| 4b | 31.753 |

| | |
|---|---|
| 4 tag | 31.328 |
| 4b tag | 31.623 |

### Example 2

Method for measuring RNA and protein in the same cell(s) with same readout or just sequencing readout (both are useful, in particular when it is not necessary to split the sample).

Cells are to be stained with antibody-DNA conjugates. The cells can be fixed or non-fixed cells. Cells can be sorted into single cells or single cell populations.

Reverse transcription is performed using polydT primer and/or random-mer for priming (standard methods can be used). For qPCR, perform multiplex QPCR with taqman reagents for both cDNAs of interest and for DNA conjugated to antibody.

When measuring protein and RNA in the same sample, there are multiple methods that can be used based on which RNA sequencing library generation method is being used. Examples:
1. Clontech SMARTer kit (with or without fluidigm C1). stain cells with DNA conjugated antibodies where 3' end of DNA has been blocked, perform cDNA reaction with clontech SMARTer kit. Selectively amplify cDNA then use Illumina tagmentation to generate RNAseq library. Amplify both the RNAseq library AND the DNA conjugated to the antibody to add a sample barcode and prepare library for sequencing. For this process, it is important that the DNA conjugated to the antibody is single stranded so that it does not react with the tagmentation reaction
2. Other RNAseq library including those used in droplets where sample barcode is added at the cDNA generation step. We will add a polyA tail to the DNA-antibody conjugates either by oligo synthesis or by polyA polymerase. Stain cells with DNA conjugated antibodies. Perform RT with oligo-dT which contains a sample barcode. Generate first and second strand cDNA (also creates a double stranded DNA conjugated antibody). Shear DNA and ligate on sequencing adaptors to generate sequenceable library. The key is that the DNA conjugated to the antibody is treated just like the RNA.
3. Amplify specific loci in the cDNA (such as gene fusion junctions or T-cell receptor loci) for sequencing similar to gDNA amplification described above
4. DNA+RNA+Protein. Basically the same as above, but include a DNA based PCR step that adds illumina adaptors to the genomic DNA amplicon - the DNA seq part is amplicon sequencing.
5. RNA readout using golden gate assay, molecular inversion probes or proximity ligation (e.g., temp-o-seq/ligation chain reaction) In this case, probes hybridizing to RNA or cDNA can have the same illumina handle sequences as the antibody-oligo conjugates or different. Different is better so that two different PCRs can be conducted, allowing for control over the amount of RNA vs protein signal that gets sequenced.

### Example 3

### Materials and Methods

The following materials and methods were used in the experiments described in FIGS. 11A, 11B, 12A-12C and 13 herein.

Cell staining: 1 million human peripheral blood mononuclear cells (PBMCs) were stained with a pool of DNA encoded antibodies. All steps took place on ice. Cells were resuspended in a blocking buffer containing the following: 10 % v/v Rabbit serum, 2 % BSA, 1 mg/ml SS salmon sperm DNA, 0.2 mg/ml Cysteine for 10 min. Cells were spun down and blocking buffer was replaced with blocking buffer containing the DNA encoded antibody pool; each antibody was at an empirically determined best concentration. Antibodies were stained for 30 min, then spun down and the antibody containing buffer was replaced with PBS+0.1%BSA. Three successive washes in the PBS+BSA buffer were performed and the cells were then used as input into the 10X genomics' chromium assays described. 10X genomics assays were performed following the manufacturer's protocol with one exception. Following the cDNA amplification step and first step of Ampure based size selection, the Ampure solution was kept instead of being discarded as per the protocol. All other steps of the protocol were followed for generation of the RNA and/or TCR sequencing libraries and suggested sequencing and analysis methods were used.

For the antibody conjugate, the saved Ampure solution was size selected by adding 2.6X the original volume of new Ampure beads. The Ampure protocol for size selection was followed to obtain purified antibody conjugated oligos (and their copies). These oligos were then PCR amplified using the following oligos:
AATGATACGGCGACCACCGAGATCTACACTCTTTCCCTACACGACGCTCTTCCGA T*C*T (SEQ ID NO:7); and
CAAGCAGAAGACGGCATACGAGATXXXXXXXXGTGACTGGAGTTCAGACGTGT GCTCTTC (SEQ ID NO:8), where XXXXXXXX represents an Illumina index read sequence. Amplifications were performed for differing numbers of cycles depending on the number of cells collected, the assay performed and the number of antibodies present in the pools.

The conjugate oligos had the following sequences: For the 3' RNA assay GACTGGAGTTCAGACGTGTGCTCTTCCGATCTZZZZZZZZNBAAAAAAAAA AAAAAAAAAAAAAAAAAA (SEQ ID NO:9), where ZZZZZZZ represents a unique antibody barcode.

For the TCR assay:
GACTGGAGTTCAGACGTGTGCTCTTCCGATCTZZZZZZZZCCCATATAAGAAA (SEQ ID NO: 10), where ZZZZZZZZ represents the unique antibody barcode.

### Data processing:

Antibody Barcodes: Cell barcodes, Unique molecular identifiers (UMIs) and Antibody barcodes were extracted from raw fastq files with custom scripts. Antibody barcodes were clustered with an edit distance of 1. Cell barcodes were clustered with an edit distance of 3. Read counts were collapsed so that each combination of cell barcode, UMI and antibody barcode was counted only once. Cells with low number of UMI reads were filtered out. This generated a cell-antibody UMI count matrix which is subsequently used for further downstream analysis. These matrices were further processed to normalize for sequencing depth or centered transformed to convert counts into a z-score. Principle component analysis was then performed and the transformed values used for t-distributed stochastic neighborhood embedding (t-SNE analysis as described in Van der Maaten, L.J.P. Accelerating t-SNE using Tree-Based Algorithms. Journal of Machine Learning Research 15, 3221-3245 (2014)). This matrix was also converted into a FCS file visualization with the FlowJo software.

RNA counts: RNA counts were generated by the 10X Genomics Cell Ranger pipeline (v 2.1.1) to generate gene-barcode matrices. Reads were mapped to the hg38 reference genome. The count data was extracted and log transformed, centered and scaled. PCA is performed and transformed values are used as input for t-SNE analysis.

Combined Protein and RNA analysis: Cell barcodes identified from both Antibody and RNA UMI counts were used for comparing protein and mRNA expression.

Antibody-based clustering: t-SNE projection was performed on antibody counts and then RNA expression values were overlayed.

Transcriptome-based clustering: t-SNE projection was performed on transcriptome counts and then protein expression based on antibody barcode values were overlayed.

As used herein, the indefinite articles "a" and "an" should be understood to mean "at least one" unless clearly indicated to the contrary.

The phrase "and/or", as used herein, should be understood to mean "either or both" of the elements so conjoined, i.e., elements that are conjunctively present in some cases and disjunctively present in other cases.

It should also be understood that, unless clearly indicated to the contrary, in any methods described herein that include more than one step or act, the order of the steps or acts of the method is not necessarily limited to the order in which the steps or acts of the method are recited.

Unless otherwise indicated or otherwise evident from the context and understanding of one of ordinary skill in the art, values that are expressed as ranges can assume any specific value or subrange within the stated ranges in various embodiments, unless the context clearly dictates otherwise.

## Claims

1. A method of detecting a target non-nucleic acid molecule and a target nucleic acid molecule in a sample, comprising the steps of:
a) contacting a sample comprising a target non-nucleic acid molecule and a target nucleic acid molecule with a binding agent under conditions that permit binding between the binding agent and the target non-nucleic acid molecule in the sample, wherein the binding agent is covalently conjugated to an oligonucleotide that comprises a binding agent identification tag and a poly(A) sequence;
b) reverse transcribing the conjugated oligonucleotide and the target nucleic acid molecule in the sample simultaneously, thereby producing a conjugated oligonucleotide reverse transcription product and a target nucleic acid molecule reverse transcription product, wherein reverse transcription of the conjugated oligonucleotide is primed using an extension primer that comprises a poly(dT) sequence, wherein the length of the poly(dT) sequence is greater than or equal to the length of the poly(A) sequence in the conjugated oligonucleotide;
c) amplifying the conjugated oligonucleotide reverse transcription product and the target nucleic acid molecule reverse transcription product simultaneously, thereby producing a conjugated oligonucleotide amplicon and a target nucleic acid molecule amplicon; and
d) detecting:
1) the conjugated oligonucleotide amplicon, thereby detecting the target non-nucleic acid molecule in the sample; and
2) the target nucleic acid molecule amplicon, thereby detecting the target nucleic molecule in the sample.

2. The method of Claim 1, wherein the binding agent is an antibody, optionally a monoclonal antibody.

3. The method of Claim 1 or 2, wherein the conjugated oligonucleotide is a DNA molecule, optionally wherein the DNA molecule is single stranded.

4. The method of any one of Claims 1-3, wherein the 3' end of the oligonucleotide is conjugated to the binding agent.

5. The method of any one of Claims 1-3, wherein:
- the 5' end of the oligonucleotide is conjugated to the binding agent;
and/or
- step b) further comprises extending the 3' end of the conjugated oligonucleotide with a reverse transcriptase enzyme to thereby produce a double stranded conjugated oligonucleotide reverse transcription product.

6. The method of any of the preceding claims, wherein the conjugated oligonucleotide further comprises
- an affinity tag; and/or
- a blocking group that prevents extension of the oligonucleotide by a polymerase, optionally wherein the blocking group is a ddNTP or an inverted dTTP, optionally wherein the blocking group is at the 3' end of the conjugated oligonucleotide.

7. The method of any of the preceding claims, wherein:
- reverse transcription of the conjugated oligonucleotide in step b) is primed using an extension primer that comprises a cell identification tag or a unique molecular identifier (UMI) sequence, or a combination thereof; and/or
- step b) further comprises reverse transcribing non-target nucleic acid molecules in the sample, thereby producing non-target nucleic acid molecule reverse transcription products, optionally wherein:
- the non-target nucleic acid molecules in the sample are non-target mRNA molecules;
- the target nucleic acid molecule reverse transcription product is selectively amplified in step c); or
- the target nucleic acid molecule reverse transcription product and the non-target nucleic acid molecule reverse transcription products are amplified in step c).

8. The method of any of the preceding claims, wherein:
- the conjugated oligonucleotide amplicon and the target nucleic acid molecule amplicon are separated prior to detecting in step d), optionally wherein the conjugated oligonucleotide amplicon and the target nucleic acid molecule amplicon are separated based on size; and/or
- the method further comprises adding binding sites for sequencing primers to the amplicons generated in step c), optionally wherein the binding sites for sequencing primers are added using transposons or by fragmenting nucleic acid molecules in the amplicons and ligating the binding sites to the fragments, by a primer extension reaction, or by a nucleic acid amplification reaction, or a combination thereof.

9. The method of any of the preceding claims, wherein:
- the conjugated oligonucleotide amplicon and the target nucleic acid amplicon are detected based on size;
- the conjugated oligonucleotide amplicon and the target nucleic acid amplicon are differentially labeled, and the detection in step d) is performed by detecting the different labels on the amplicons;
- detecting the conjugated oligonucleotide amplicon and the target nucleic acid amplicon in step d) comprises sequencing the amplicons; and/or
- the method further comprises quantifying expression of the target non-nucleic acid molecule, the target nucleic acid molecule, or both, in the sample.

10. The method of any of the preceding claims, wherein:
- the target non-nucleic acid molecule is a target protein;
- the target nucleic acid molecule is a target RNA molecule, optionally wherein the target RNA molecule is a target mRNA molecule;
- the sample comprises a plurality of cells, optionally wherein the method further comprises isolating single cells from the sample comprising a plurality of cells prior to step b); and/or
- the sample comprises a single cell.

11. The method of any of the preceding claims, wherein the extension primer comprises the poly(T) sequence at its 3' end.

12. A method of detecting a target protein and a target nucleic acid molecule in a sample according to claim 1, comprising the steps of:
a) contacting a sample comprising a target protein and a target nucleic acid molecule with a binding agent under conditions that permit binding between the binding agent and the target protein in the sample, wherein the binding agent is covalently conjugated to an oligonucleotide that comprises a binding agent identification tag and a poly(A) sequence;
b) reverse transcribing the conjugated oligonucleotide and the target nucleic acid molecule in the sample simultaneously, thereby producing a conjugated oligonucleotide reverse transcription product and a target nucleic acid molecule reverse transcription product, wherein reverse transcription of the conjugated oligonucleotide is primed using an extension primer that comprises a poly(dT) sequence, wherein the length of the poly(dT) sequence is greater than or equal to the length of the poly(A) sequence in the conjugated oligonucleotide;
c) amplifying the conjugated oligonucleotide reverse transcription product and the target nucleic acid molecule reverse transcription product simultaneously, thereby producing a first conjugated oligonucleotide amplicon and a first target nucleic acid molecule amplicon;
d) separating the first conjugated oligonucleotide amplicon and the first target nucleic acid molecule amplicon;
e) amplifying the separated conjugated oligonucleotide amplicon and target nucleic acid molecule amplicon in separate reactions, thereby producing a second conjugated oligonucleotide amplicon and a second target nucleic acid molecule amplicon; and
f) detecting:
1) the second conjugated oligonucleotide amplicon, thereby detecting the target protein in the sample; and
2) the second target nucleic acid molecule amplicon, thereby detecting the target nucleic molecule in the sample.

## Patentansprüche

1. Verfahren zum Nachweisen eines Nicht-Nukleinsäure-Zielmoleküls und eines Nukleinsäure-Zielmoleküls in einer Probe, umfassend:
a) In-Kontakt-Bringen einer ein Nicht-Nukleinsäure-Zielmolekül und ein Nukleinsäure-Zielmolekül umfassenden Probe mit einem Bindemittel unter Bedingungen, die eine Bindung zwischen dem Bindemittel und dem Nicht-Nukleinsäure-Zielmolekül in der Probe gestatten, wobei das Bindemittel kovalent an ein Oligonukleotid konjugiert ist, das ein Bindemittelidentifikations-Tag und eine Poly(A)-Sequenz umfasst;
b) gleichzeitiges Revers-Transkribieren des konjugierten Oligonukleotids und des Nukleinsäure-Zielmoleküls in der Probe, wodurch ein Konjugiertes-Oligonukleotid-Reverse-Transkriptionsprodukt und ein Nukleinsäure-Zielmolekül-Reverse-Transkriptionsprodukt erzeugt werden, wobei das Priming der reversen Transkription des konjugierten Oligonukleotids unter Verwendung eines Verlängerungsprimers erfolgt, der eine Poly(dT)-Sequenz umfasst, wobei die Poly(dT)-Sequenz länger als oder gleichlang wie die Poly(A)-Sequenz im konjugierten Oligonukleotid ist;
c) gleichzeitiges Amplifizieren des Konjugiertes-Oligonukleotid-Reverse-Transkriptionsprodukts und des Nukleinsäure-Zielmolekül-Reverse-Transkriptionsprodukts, wodurch ein Konjugiertes-Oligonukleotid-Amplifikat und ein Nukleinsäure-Zielmolekül-Amplifikat erzeugt werden; und
d) Nachweisen
1) des Konjugiertes-Oligonukleotid-Amplifikats, wodurch das Nicht-Nukleinsäure-Zielmolekül in der Probe nachgewiesen wird; und
2) des Nukleinsäure-Zielmolekül-Amplifikats, wodurch das Zielnukleinmolekül in der Probe nachgewiesen wird.

2. Verfahren nach Anspruch 1, wobei es sich bei dem Bindemittel um einen Antikörper, gegebenenfalls einen monoklonalen Antikörper handelt.

3. Verfahren nach Anspruch 1 oder 2, wobei es sich bei dem konjugierten Oligonukleotid um ein DNA-Molekül handelt, gegebenenfalls wobei das DNA-Molekül als Einzelstrang vorliegt.

4. Verfahren nach einem der Ansprüche 1-3, wobei das 3'-Ende des Oligonukleotids an das Bindemittel konjugiert ist.

5. Verfahren nach einem der Ansprüche 1-3, wobei:
- das 5'-Ende des Oligonukleotids an das Bindemittel konjugiert ist; und/oder
- Schritt b) ferner Verlängern des 3'-Endes des konjugierten Oligonukleotids mit einem Reverse-Transkriptase-Enzym umfasst, wodurch ein doppelsträngiges Konjugiertes-Oligonukleotid-Reverse-Transkriptionsprodukt erzeugt wird.

6. Verfahren nach einem der vorhergehenden Ansprüche, wobei das konjugierte Oligonukleotid ferner
- ein Affinitäts-Tag umfasst; und/oder
- eine Blockergruppe umfasst, die die Verlängerung des Oligonukleotids durch eine Polymerase verhindert, gegebenenfalls wobei es sich bei der Blockergruppe um ein ddNTP oder ein invertiertes dTTP handelt, gegebenenfalls wobei sich die Blockergruppe am 3'-Ende des konjugierten Oligonukleotids befindet.

7. Verfahren nach einem der vorhergehenden Ansprüche, wobei:
- das Priming der reversen Transkription des konjugierten Oligonukleotids in Schritt b) unter Verwendung eines Verlängerungsprimers erfolgt, der ein Zellidentifikations-Tag oder eine UMI(eindeutiger Inhaltsstoff-Identifikator)-Sequenz oder eine Kombination davon umfasst;
und/oder
- Schritt b) ferner Revers-Transkribieren von Nukleinsäure-Nicht-Zielmolekülen in der Probe umfasst, wodurch Nukleinsäure-Nicht-Zielmolekül-Reverse-Transkriptionsprodukte erzeugt werden, gegebenenfalls wobei:
- es sich bei den Nukleinsäure-Nicht-Zielmolekülen in der Probe um mRNA-Nicht-Zielmoleküle handelt;
- das Nukleinsäure-Zielmolekül-Reverse-Transkriptionsprodukt in Schritt c) selektiv amplifiziert wird; oder
- das Nukleinsäure-Zielmolekül-Reverse-Transkriptionsprodukt und das Nukleinsäure-Nicht-Zielmolekül-Reverse-Transkriptionsprodukt in Schritt c) amplifiziert werden.

8. Verfahren nach einem der vorhergehenden Ansprüche, wobei:
- das Konjugiertes-Oligonukleotid-Amplifikat und das Nukleinsäure-Zielmolekül-Amplifikat vor dem Nachweisen in Schritt d) getrennt werden, gegebenenfalls wobei das Konjugiertes-Oligonukleotid-Amplifikat und das Nukleinsäure-Zielmolekül-Amplifikat nach Größe getrennt werden;
und/oder
- das Verfahren ferner Hinzufügen von Bindungsstellen für Sequenzierprimer zu den in Schritt c) erzeugten Amplifikaten umfasst, gegebenenfalls wobei die Bindungsstellen für Sequenzierprimer unter Verwendung von Transposons oder durch Fragmentieren von Nukleinsäuremolekülen in den Amplifikaten und Ligieren der Bindungsstellen an die Fragmente, durch eine Primerverlängerungsreaktion oder durch eine Nukleinsäureamplifikationsreaktion oder eine Kombination davon hinzugefügt werden.

9. Verfahren nach einem der vorhergehenden Ansprüche, wobei:
- das Konjugiertes-Oligonukleotid-Amplifikat und das Nukleinsäure-Zielmolekül-Amplifikat anhand der Größe nachgewiesen werden;
- das Konjugiertes-Oligonukleotid-Amplifikat und das Nukleinsäure-Zielmolekül-Amplifikat unterschiedlich markiert sind und der Nachweis in Schritt d) über Nachweisen der verschiedenen Markierungen auf den Amplifikaten erfolgt;
- Nachweisen des Konjugiertes-Oligonukleotid-Amplifikats und des Nukleinsäure-Zielmolekül-Amplifikats in Schritt d) Sequenzieren der Amplifikate umfasst; und/oder
- das Verfahren ferner Quantifizieren der Expression des Nicht-Nukleinsäure-Zielmoleküls oder bzw. und des Nukleinsäure-Zielmoleküls in der Probe umfasst.

10. Verfahren nach einem der vorhergehenden Ansprüche, wobei:
- es sich bei dem Nicht-Nukleinsäure-Zielmolekül um ein Zielprotein handelt;
- es sich bei dem Nukleinsäure-Zielmolekül um ein RNA-Zielmolekül handelt, gegebenenfalls wobei es sich bei dem RNA-Zielmolekül um ein mRNA-Zielmolekül handelt;
- die Probe mehrere Zellen umfasst, gegebenenfalls wobei das Verfahren ferner Isolieren von Einzelzellen aus der mehrere Zellen umfassenden Probe von Schritt b) umfasst; und/oder
- die Probe eine Einzelzelle umfasst.

11. Verfahren nach einem der vorhergehenden Ansprüche, wobei der Verlängerungsprimer die Poly(dT)-Sequenz an seinem 3'-Ende umfasst.

12. Verfahren zum Nachweisen eines Zielproteins und eines Nukleinsäure-Zielmoleküls in einer Probe gemäß Anspruch 1, umfassend die Schritte:
a) In-Kontakt-Bringen einer ein Zielprotein und ein Nukleinsäure-Zielmolekül umfassenden Probe mit einem Bindemittel unter Bedingungen, die eine Bindung zwischen dem Bindemittel und dem Zielprotein in der Probe gestatten, wobei das Bindemittel kovalent an ein Oligonukleotid konjugiert ist, das ein Bindemittelidentifikations-Tag und eine Poly(A)-Sequenz umfasst;
b) gleichzeitiges Revers-Transkribieren des konjugierten Oligonukleotids und des Nukleinsäure-Zielmoleküls in der Probe, wodurch ein Konjugiertes-Oligonukleotid-Reverse-Transkriptionsprodukt und ein Nukleinsäure-Zielmolekül-Reverse-Transkriptionsprodukt erzeugt werden, wobei das Priming der reversen Transkription des konjugierten Oligonukleotids unter Verwendung eines Verlängerungsprimers erfolgt, der eine Poly(dT)-Sequenz umfasst, wobei die Poly(dT)-Sequenz länger als oder gleichlang wie die Poly(A)-Sequenz im konjugierten Oligonukleotid ist;
c) gleichzeitiges Amplifizieren des Konjugiertes-Oligonukleotid-Reverse-Transkriptionsprodukts und des Nukleinsäure-Zielmolekül-Reverse-Transkriptionsprodukts, wodurch ein erstes Konjugiertes-Oligonukleotid-Amplifikat und ein erstes Nukleinsäure-Zielmolekül-Amplifikat erzeugt werden; und
d) Trennen des ersten Konjugiertes-Oligonukleotid-Amplifikats und des ersten Nukleinsäure-Zielmolekül-Amplifikats;
e) Amplifizieren des getrennten Konjugiertes-Oligonukleotid-Amplifikats und Nukleinsäure-Zielmolekül-Amplifikats in getrennten Reaktionen, wodurch ein zweites Konjugiertes-Oligonukleotid-Amplifikat und ein zweites Nukleinsäure-Zielmolekül-Amplifikat erzeugt werden; und
f) Nachweisen
1) des zweiten Konjugiertes-Oligonukleotid-Amplifikats, wodurch das Zielprotein in der Probe nachgewiesen wird; und
2) des zweiten Nukleinsäure-Zielmolekül-Amplifikats, wodurch das Zielnukleinmolekül in der Probe nachgewiesen wird.

## Revendications

1. Procédé de détection d'une molécule d'acide non nucléique cible et d'une molécule d'acide nucléique cible dans un échantillon, comprenant les étapes de :
a) mise en contact d'un échantillon contenant une molécule d'acide non nucléique cible et une molécule d'acide nucléique cible avec un agent de liaison dans des conditions qui permettent une liaison entre l'agent de liaison et la molécule d'acide non nucléique cible dans l'échantillon, l'agent de liaison étant conjugué par liaison covalente à un oligonucléotide qui comprend une étiquette d'identification de l'agent de liaison et une séquence poly(A) ;
b) transcription inverse simultanée de l'oligonucléotide conjugué et de la molécule d'acide nucléique cible, en produisant ainsi un produit de transcription inverse d'oligonucléotide conjugué et un produit de transcription inverse de molécule d'acide nucléique cible, la transcription inverse de l'oligonucléotide conjugué étant amorcée par utilisation d'une amorce d'extension qui comprend une séquence poly(dT), la longueur de la séquence poly(dT) étant supérieure ou égale à la longueur de la séquence poly(A) dans l'oligonucléotide conjugué ;
c) amplification simultanée du produit de transcription inverse d'oligonucléotide conjugué et du produit de transcription inverse de molécule d'acide nucléique cible, en produisant ainsi un amplicon d'oligonucléotide conjugué et un amplicon de molécule d'acide nucléique cible ; et
d) détection
1) de l'amplicon d'oligonucléotide conjugué, pour détecter ainsi la molécule d'acide non nucléique cible dans l'échantillon ; et
2) de l'amplicon de molécule d'acide nucléique cible, pour détecter ainsi la molécule d'acide nucléique cible dans l'échantillon.

2. Procédé selon la revendication 1, dans lequel l'agent de liaison est un anticorps, éventuellement un anticorps monoclonal.

3. Procédé selon la revendication 1 ou 2, dans lequel l'oligonucléotide conjugué est une molécule d'ADN, éventuellement dans lequel la molécule d'ADN est simple brin.

4. Procédé selon l'une quelconque des revendications 1 à 3, dans lequel l'extrémité 3' de l'oligonucléotide est conjuguée à l'agent de liaison.

5. Procédé selon l'une quelconque des revendications 1 à 3, dans lequel
- l'extrémité 5' de l'oligonucléotide est conjuguée à l'agent de liaison ;
et/ou
- l'étape b) comprend en outre l'extension de l'extrémité 3' de l'oligonucléotide conjugué avec une enzyme transcriptase inverse pour produire ainsi un produit de transcription inverse d'oligonucléotide conjugué double brin.

6. Procédé selon l'une quelconque des revendications précédentes, dans lequel l'oligonucléotide conjugué comprend en outre
- une étiquette d'affinité ; et/ou
- un groupe bloquant qui empêche l'extension de l'oligonucléotide par une polymérase, éventuellement dans lequel le groupe bloquant est un ddNTP ou un dTTP inversé, éventuellement dans lequel le groupe bloquant se trouve à l'extrémité 3' de l'oligonucléotide conjugué.

7. Procédé selon l'une quelconque des revendications précédentes, dans lequel
- la transcription inverse de l'oligonucléotide conjugué de l'étape b) est amorcée par utilisation d'une amorce d'extension qui comprend une étiquette d'identification de cellules ou une séquence d'identifiant moléculaire unique (UMI), ou une combinaison de celles-ci ; et/ou
- l'étape b) comprend en outre la transcription inverse de molécules d'acide nucléique non-cible dans l'échantillon, pour produire ainsi des produits de transcription inverse de molécule d'acide nucléique non-cible, éventuellement dans laquelle :
- les molécules d'acide nucléique non-cible de l'échantillon sont des molécules d'ARNm non-cible ;
- le produit de transcription inverse de molécule d'acide nucléique cible est sélectivement amplifié dans l'étape c) ; ou
- le produit de transcription inverse de molécule d'acide nucléique cible et les produits de transcription inverse de molécule d'acide nucléique non-cible sont amplifiés dans l'étape c).

8. Procédé selon l'une quelconque des revendications précédentes, dans lequel
- l'amplicon d'oligonucléotide conjugué et l'amplicon de molécule d'acide nucléique cible sont séparés avant la détection de l'étape d), éventuellement dans lequel l'amplicon d'oligonucléotide conjugué et l'amplicon de molécule d'acide nucléique cible sont séparés en fonction de leur taille ; et/ou
- le procédé comprenant en outre l'addition de sites de liaison pour d'amorces de séquençage aux amplicons générés dans l'étape c), éventuellement dans lequel les sites de liaison pour amorces de séquençage sont ajoutés par utilisation de transposons ou par fragmentation de molécules d'acide nucléique dans les amplicons et ligature des sites de liaison aux fragments, par une réaction d'extension d'amorce ou par une réaction d'amplification d'acide nucléique, ou une combinaison de celles-ci.

9. Procédé selon l'une quelconque des revendications précédentes, dans lequel
- l'amplicon d'oligonucléotide conjugué et l'amplicon d'acide nucléique cible sont détectés en fonction de leur taille ;
- l'amplicon d'oligonucléotide conjugué et l'amplicon d'acide nucléique cible sont marqués d'une manière différentielle, et la détection de l'étape d) est mise en oeuvre par détection des marqueurs différents se trouvant sur les amplicons ;
- la détection de l'amplicon d'oligonucléotide conjugué et de l'amplicon d'acide nucléique cible de l'étape d) comprennent le séquençage des amplicons ; et/ou
- le procédé comprend en outre la quantification de l'expression de la molécule d'acide non nucléique cible, de la molécule d'acide nucléique cible, ou des deux, dans l'échantillon.

10. Procédé selon l'une quelconque des revendications précédentes, dans lequel
- la molécule d'acide non nucléique cible est une protéine cible ;
- la molécule d'acide nucléique cible est une molécule d'ARN cible, éventuellement dans lequel la molécule d'ARN cible est une molécule d'ARNm cible ;
- l'échantillon comprend une pluralité de cellules, éventuellement dans lequel le procédé comprend en outre avant l'étape b) l'isolement de cellules uniques de l'échantillon comprenant une pluralité de cellules ; et/ou
- l'échantillon comprend une cellule unique.

11. Procédé selon l'une quelconque des revendications précédentes, dans lequel l'amorce d'extension comprend la séquence poly(T) en son extrémité 3'.

12. Procédé de détection d'une protéine cible et d'une molécule d'acide nucléique cible dans un échantillon selon la revendication 1, comprenant les étapes de :
a) mise en contact d'un échantillon contenant une protéine cible et une molécule d'acide nucléique cible avec un agent de liaison dans des conditions qui permettent une liaison entre l'agent de liaison et la protéine cible dans l'échantillon, l'agent de liaison étant conjugué par liaison covalente à un oligonucléotide qui comprend une étiquette d'identification de l'agent de liaison et une séquence poly(A) ;
b) transcription inverse simultanée de l'oligonucléotide conjugué et de la molécule d'acide nucléique cible, en produisant ainsi un produit de transcription inverse d'oligonucléotide conjugué et un produit de transcription inverse de molécule d'acide nucléique cible, la transcription inverse de l'oligonucléotide conjugué étant amorcée par utilisation d'une amorce d'extension qui comprend une séquence poly(dT), la longueur de la séquence poly(dT) étant supérieure ou égale à la longueur de la séquence poly(A) dans l'oligonucléotide conjugué ;
c) amplification simultanée du produit de transcription inverse d'oligonucléotide conjugué et du produit de transcription inverse de molécule d'acide nucléique cible, en produisant ainsi un premier amplicon d'oligonucléotide conjugué et un premier amplicon de molécule d'acide nucléique cible ;
d) la séparation du premier amplicon d'oligonucléotide conjugué et du premier amplicon de molécule d'acide nucléique cible ;
e) l'amplification de l' amplicon d'oligonucléotide conjugué et de l' amplicon de molécule d'acide nucléique cible séparés dans des réactions distinctes, pour produire ainsi un second amplicon d'oligonucléotide conjugué et un second amplicon de molécule d'acide nucléique cible ; et
f) la détection :
1) du second amplicon d'oligonucléotide conjugué, pour détecter ainsi la protéine cible dans l'échantillon ; et
2) du second amplicon de molécule d'acide nucléique cible, pour détecter ainsi la molécule d'acide nucléique cible dans l'échantillon.
